# EUROPEAN PATENT APPLICATION

(11) **EP 4 286 410 A1**
(43) Date of publication of application: **06.12.2023**
(21) Application number: 22745189.5
(22) Date of filing: 24.01.2022
(51) Int. Cl.: C07K 16/28, A61P 35/00

(54) **SINGLE-DOMAIN ANTIBODY AGAINST CD16A AND USE THEREOF**

(30) Priority: 27.01.2021 CN 202110112841
(71) Applicant: Innovent Biologics (Suzhou) Co., Ltd., Suzhou, Jiangsu 215123 (CN)
(72) Inventor: HU, Siyi, Suzhou, Jiangsu 215123 (CN); ZHOU, Shuaixiang, Suzhou, Jiangsu 215123 (CN)
(74) Representative: Sagittarius IP
(86) International application number: PCT/CN2022/073488
(87) International publication number: WO 2022/161314

(57) **Abstract**

The present invention relates to an isolated anti-CD16A single-domain antibody, and a monospecific antibody molecule or a multispecific antibody molecule comprising the anti-CD16A single-domain antibody as a moiety. The present invention also relates to a nucleic acid encoding the anti-CD16A single-domain antibody, the monospecific antibody molecule, or the multispecific antibody molecules, a host cell comprising the nucleic acid, and a method for preparing the anti-CD16A single-domain antibody, the monospecific antibody molecule, or the multispecific antibody molecule. Furthermore, the present invention relates to use of the anti-CD16A single-domain antibody, the monospecific antibody molecule, or the multispecific antibody molecule for the treatment and/or prevention or diagnosis of acute and chronic infectious diseases (e.g., bacterial or viral infections) or a tumor.

## Description

### TECHNICAL FIELD

The present invention generally relates to an antibody and use thereof. More specifically, the present invention relates to a single-domain antibody against CD16A, and a monospecific antibody molecule or a multispecific antibody molecule comprising same. The present invention also relates to use thereof for the treatment and/or prevention or diagnosis of acute and chronic infectious diseases (e.g., bacterial or viral infections) or a tumor.

### BACKGROUND

Natural killer cells (NKs) are a large class of innate immune cells present in humans that are involved in early defense. Unlike innate immune cells (e.g., CD8⁺ T cells or CD4⁺ T cells), NK cells tend to exist in a constitutively activated state and can directly kill exogenous microorganisms, virus-infected cells, or tumor cells without the need for antigen presentation or prior sensitization with histocompatibility complexes (MHCs). Therefore, among all immune effector cells, the NK cell is one of the relatively attractive candidates for immunotherapy. To date, bispecific antibodies against CD16/EGFR, CD16/CD30, or the like, have been developed (Kristina Ellwanger et al., MAbs, 2019, 11(5), 899-918; Uwe Reusch et al., Mabs, 2014, 6:3, 727-738).

Antibody-dependent cell-mediated cytotoxicity (ADCC) is one of the major mechanisms by which NK cells mediate killing of target and foreign host cells. By binding of an Fc region of an antibody to an Fc receptor FcyRIIIA (CD16A) expressed on NK cells, the NK cells are activated to exert the ADCC effect. Bispecific antibodies that generate antigen-dependent ADCC by recruiting NK cells to redirect target tumor cell antigens are receiving increasing attention from researchers. However, the affinity of CD16A expressed on NK cells for the Fc region of different antibodies varies, leading to variable ADCC effects. Therefore, it is desirable to develop high-affinity antibodies that specifically bind to CD16A to enhance the ADCC effect.

To date, tetravalent bispecific antibodies based on NK effector cells developed by Affimed Therapeutics AG, i.e., anti-CD16A/CD30 antibody (also referred to as "AFM13") and anti-CD16A/EGFR antibody (also referred to as "AFM24") TandAbs, have been subjected to phase II and phase I clinical trials, respectively, for the treatment of Hodgkin's lymphoma and a solid cancer with positive EGFR expression, respectively. AFM13 TandAb and AFM24 TandAb from Affimed Therapeutics AG activate NK cells to mediate ADCC activity based primarily on their specific targeting of an antibody fragment (also referred to as "4LS21 scFv") of an activated receptor CD16A on NK cells, thereby killing target tumor cells. The 4LS21 scFv binds only to CD16A on NK cells and not to CD16B on neutrophils or polymorphonuclear granulocytes. Also, due to a 4LS21 scFv-specific antigen-binding epitope (CD16A-Y140), the 4LS21 scFv does not compete with a large amount of IgG in plasma for binding to CD16A. Therefore, its therapeutic activity is not affected by irrelevant IgG. In addition, the 4LS21 scFv-specific binding epitope (CD16A-Y140) determines that its therapeutic activity is independent of the CD16A subtype (158V or 158F), avoiding the preference of therapeutic antibody-mediated ADCC for different patients.

CD16A (i.e., FcyRIIIA) is a low-affinity and dominant activated transmembrane receptor expressed on NK cells, macrophages, and mastocytes, and is a member of transmembrane receptors of the immunoglobulin superfamily. On NK cells, the α chain of FcyRIIIA binds to an immunoreceptor tyrosine activation motif (ITAM) containing an FcεRIγ chain and/or a T cell receptor (TCR)/CD3ζ chain for signal transduction (Wirthmueller et al., 1992, J. Exp. Med. 175: 1381-1390). According to the difference between allelic polymorphisms at position 158 at the N terminus of CD16A, the differential expression of valine or phenylalanine at position 158 of CD16A exists, so that subtypes of CD16A-158V/V (about 15%), CD16A-158V/F (about 25%), and CD16A-158F/F (about 60%) exist in the population. The affinity values of different CD16A subtypes for IgG are different by 5-10 times. Among these subtypes, type 158V/V has the strongest affinity, and therefore, the therapeutic antibody against the type has a best clinical ADCC effect.

CD16B is a glycosyl-phosphatidylinositol (GPI)-anchored transmembrane signal receptor whose extracellular domain has 96% amino acid homology to CD16A, and comprises three different subtypes, i.e., NA1, NA2, and SH. Asians are mainly of type NA1 (about 68%), Caucasians are mainly of type NA2 (about 65%), and SH is mainly present in Caucasians and Africans, accounting for about 2.5%.

At the cellular level, CD16B is almost exclusively expressed in polymorphonuclear granulocytes (PMNs), generally has the number of expressed copies per cell of 100,000 to 300,000 copies/cell, and plays an important role in the survival and degranulation of polymorphonuclear granulocytes. Meanwhile, there is a large amount of free CD16B generated by the enzymolysis of metalloprotease ADAM17 in serum, which is chelated with antibodies in serum to form a complex, resulting in serious adverse reactions.

Therefore, it is particularly necessary to develop a high-affinity and CD16A subtype-independent CD16A antibody that specifically binds to CD16A and does not bind to CD16B.

The CD16A humanized antibody 4LS21 from Affimed Therapeutics AG combines the advantages. It not only enlarges the population of drug administration, but also greatly improves the selectivity of the effect of NK cells. In addition, it does not compete with IgG in serum, and can effectively avoid unpredictable adverse reactions caused by binding to CD16B on neutrophils.

However, the 4LS21-based TandAb bispecific antibody is in the form of "2 scFv domains for one antigen + 2 scFv domains for another antigen" (hereinafter also referred to as "2 + 2") with 8 binding domains (subdomains) and linked by at least 6 linkers, which presents many challenges for the expression, purification, engineering, and production processes of the TandAb bispecific antibody, as 4LS21 is a conventional antibody clone with 6 CDRs, and is generally assembled in the form of scFv during construction of the bispecific antibody, which not only increases the process difficulty, but also limits the degree of freedom in construction of the bispecific antibody, and thus is not conducive to producing the optimal bispecific antibody format to match its corresponding bioactivity mechanism. Meanwhile, given that TandAb has a relatively large molecular weight, which increases immunogenicity and makes it difficult to permeate into tumor tissues, its therapeutic effect is reduced or toxic and side effects are increased to a certain extent. In addition, given its tetravalent structure in the form of "2 + 2" linked by multiple linkers, TandAb carries the risk of easily generating non-specific activation of CD16A on NK cells, thereby inducing immeasurable adverse reactions.

A single-domain antibody (sdAb) is the smallest antibody molecule at present, with a molecular weight of 1/10 that of an intact antibody. Besides the antigen reactivity of the intact antibody, the single-domain antibody has some unique functional properties, such as low molecular weight, strong stability, good solubility, easy expression, weak immunogenicity, strong penetrability, strong targeting property, and low preparation costs, and almost overcomes the defects of conventional antibodies, such as long development cycles, low stability, and harsh storage conditions.

Therefore, it is particularly necessary to research and develop a high-affinity single-domain CD16A antibody that has a small molecular weight and good tumor permeability, can be freely assembled into a bispecific antibody, and has all the biological properties of 4LS21 (i.e., high specificity for CD16A, independence of CD16A subtype (158V or 158F), no binding to CD16B, and no competition with IgG *in vivo).*

### SUMMARY

Through extensive and intensive studies and extensive screening, the inventors have succeeded in obtaining a high-affinity single-domain antibody (sdAb) that specifically binds to human CD16A, does not bind to human CD16B, and cross-reacts with monkey CD16A. Experimental results show that the single-domain antibody can specifically bind to human CD16A, does not bind to human CD16B, and cross-reacts with monkey CD16A; meanwhile, it has relatively high binding affinity, small molecular weight and good stability, has strong antigen-specific activation of human NK cells and tumor inhibitory effect, and thus is an ideal single-domain antibody for construction of bispecific antibodies or NK cell engagers and research and development of anti-tumor active drugs.

Therefore, the present invention provides a high-affinity single-domain antibody that specifically binds to human CD16A, does not bind to human CD16B, and cross-reacts with monkey CD16A, which has the following properties:
1) capable of performing construction and assembly of multispecific antibodies (e.g., bispecific antibodies) in different structural forms in free combination with different antibodies and antibody fragments against other antigens to maximize activation of NK cells and mobilize NK cells to mediate, for example, anti-tumor immunity; and
2) overcoming problems of low tumor tissue permeability, strong immunogenicity, and the like, caused by overlarge molecular weight and steric hindrance of the conventional scFv or Fab.

Therefore, in a first aspect, the present invention provides an isolated anti-CD16A single-domain antibody, comprising:
(a) 3 CDRs in an amino acid sequence of a heavy chain variable region set forth in SEQ ID NO: 1; or a CDR variant having no more than 3, no more than 2, or no more than 1 amino acid residue replacement as compared to any one of the 3 CDRs;
(b) 3 CDRs in an amino acid sequence of a heavy chain variable region set forth in SEQ ID NO: 2; or a CDR variant having no more than 3, no more than 2, or no more than 1 amino acid residue replacement as compared to any one of the 3 CDRs;
(c) 3 CDRs in an amino acid sequence of a heavy chain variable region set forth in SEQ ID NO: 3; or a CDR variant having no more than 3, no more than 2, or no more than 1 amino acid residue replacement as compared to any one of the 3 CDRs; or
(d) 3 CDRs in an amino acid sequence of a heavy chain variable region set forth in SEQ ID NO: 4; or a CDR variant having no more than 3, no more than 2, or no more than 1 amino acid residue replacement as compared to any one of the 3 CDRs.

In some embodiments, the isolated anti-CD16A single-domain antibody of the present invention comprises:
(a) HCDR1 set forth in SEQ ID NO: 23 or a variant having no more than 3, no more than 2, or no more than 1 amino acid residue replacement of HCDR1 set forth in SEQ ID NO: 23, HCDR2 set forth in SEQ ID NO: 24 or a variant having no more than 3, no more than 2, or no more than 1 amino acid residue replacement of HCDR2 set forth in SEQ ID NO: 24, and HCDR3 set forth in SEQ ID NO: 25 or a variant having no more than 3, no more than 2, or no more than 1 amino acid residue replacement of HCDR3 set forth in SEQ ID NO: 25;
(b) HCDR1 set forth in SEQ ID NO: 26 or a variant having no more than 3, no more than 2, or no more than 1 amino acid residue replacement of HCDR1 set forth in SEQ ID NO: 26, HCDR2 set forth in SEQ ID NO: 27 or a variant having no more than 3, no more than 2, or no more than 1 amino acid residue replacement of HCDR2 set forth in SEQ ID NO: 27, and HCDR3 set forth in SEQ ID NO: 28 or a variant having no more than 3, no more than 2, or no more than 1 amino acid residue replacement of HCDR3 set forth in SEQ ID NO: 28;
(c) HCDR1 set forth in SEQ ID NO: 29 or a variant having no more than 3, no more than 2, or no more than 1 amino acid residue replacement of HCDR1 set forth in SEQ ID NO: 29, HCDR2 set forth in SEQ ID NO: 30 or a variant having no more than 3, no more than 2, or no more than 1 amino acid residue replacement of HCDR2 set forth in SEQ ID NO: 30, and HCDR3 set forth in SEQ ID NO: 31 or a variant having no more than 3, no more than 2, or no more than 1 amino acid residue replacement of HCDR3 set forth in SEQ ID NO: 31; or
(d) HCDR1 set forth in SEQ ID NO: 32 or a variant having no more than 3, no more than 2, or no more than 1 amino acid residue replacement of HCDR1 set forth in SEQ ID NO: 32, HCDR2 set forth in SEQ ID NO: 33 or a variant having no more than 3, no more than 2, or no more than 1 amino acid residue replacement of HCDR2 set forth in SEQ ID NO: 33, and HCDR3 set forth in SEQ ID NO: 34 or a variant having no more than 3, no more than 2, or no more than 1 amino acid residue replacement of HCDR3 set forth in SEQ ID NO: 34, wherein the HCDRs are numbered according to the Kabat numbering scheme.

Preferably, the isolated anti-CD16A single-domain antibody comprises:
(a) HCDR1 set forth in SEQ ID NO: 23, HCDR2 set forth in SEQ ID NO: 24, and HCDR3 set forth in SEQ ID NO: 25;
(b) HCDR1 set forth in SEQ ID NO: 26, HCDR2 set forth in SEQ ID NO: 27, and HCDR3 set forth in SEQ ID NO: 28;
(c) HCDR1 set forth in SEQ ID NO: 29, HCDR2 set forth in SEQ ID NO: 30, and HCDR3 set forth in SEQ ID NO: 31; or
(d) HCDR1 set forth in SEQ ID NO: 32, HCDR2 set forth in SEQ ID NO: 33, and HCDR3 set forth in SEQ ID NO: 34.

In some embodiments, the isolated anti-CD16A single-domain antibody of the present invention comprises:
(a) a heavy chain variable region comprising an amino acid sequence of SEQ ID NO: 1 or a sequence having at least 90%, 91%, 92%, 93%, 94%, 95%, 96%, 97%, 98%, or 99% identity thereto;
(b) a heavy chain variable region comprising an amino acid sequence of SEQ ID NO: 2 or a sequence having at least 90%, 91%, 92%, 93%, 94%, 95%, 96%, 97%, 98%, or 99% identity thereto;
(c) a heavy chain variable region comprising an amino acid sequence of SEQ ID NO: 3 or a sequence having at least 90%, 91%, 92%, 93%, 94%, 95%, 96%, 97%, 98%, or 99% identity thereto; or
(d) a heavy chain variable region comprising an amino acid sequence of SEQ ID NO: 4 or a sequence having at least 90%, 91%, 92%, 93%, 94%, 95%, 96%, 97%, 98%, or 99% identity thereto.

In a second aspect, the present invention provides a monospecific antibody molecule or an antigen-binding fragment comprising the anti-CD16A single-domain antibody moiety of the present invention and an immunoglobulin Fc region, wherein optionally, the Fc region lacks effector functions or has reduced effector functions, for example, the Fc region has a LALA mutation.

In a third aspect, the present invention provides a multispecific antibody molecule or an antigen-binding fragment comprising at least (i) the anti-CD16A single-domain antibody moiety of the present invention; (ii) a Fab fragment binding to a second antigen; and optionally (iii) an immunoglobulin Fc region at the C-termini of the (i) and (ii), wherein optionally, the Fc region lacks effector functions or has reduced effector functions, for example, the Fc region has a LALA mutation.

In one embodiment, the multispecific antibody molecule is a bispecific antibody molecule.

In some embodiments, the immunoglobulin in the monospecific antibody molecule or the antigen-binding fragment or the multispecific antibody molecule (e.g., bispecific antibody molecule) or the antigen-binding fragment of the present invention is an IgG1, IgG2, or IgG4 immunoglobulin, preferably, the immunoglobulin is an IgG 1 immunoglobulin, and more preferably the immunoglobulin is a human IgG1 immunoglobulin.

In some embodiments, the heavy chains in the monospecific antibody molecule or the antigen-binding fragment or the multispecific antibody molecule (e.g., bispecific antibody molecule) or the antigen-binding fragment of the present invention each comprise a protuberance or a cavity in their Fc domains, and the protuberance or the cavity in the Fc domain of one heavy chain can be placed in the cavity or the protuberance in the Fc domain of the other heavy chain, such that the heavy chains of the antibody molecule form a stable "knob-in-hole" association with each other.

In some embodiments, the second antigen in the multispecific antibody molecule (e.g., bispecific antibody molecule) or the antigen-binding fragment of the present invention is selected from a tumor-associated antigen, a virus-associated antigen, and a bacteria-associated antigen, wherein the tumor-associated antigen is selected from, for example, HER2, BCMA, MAGE, BAGE, MART, gp100, CD19, CD20, CD30, CD33, CD70, EpCAM, EGFR1, EGFR2, EGFR3, EGFR4, PLAP, PSMA, Claudin18.2, PD-L1, MUC1, and MUC16.

In one embodiment, the multispecific antibody molecule of the present invention is an anti-CD16A/HER2 bispecific antibody or an anti-CD 16A/BCMA bispecific antibody.

In a fourth aspect, the present invention provides an isolated nucleic acid encoding the anti-CD16A single-domain antibody, the monospecific antibody molecule or the antigen-binding fragment, or the multispecific antibody molecule (e.g., bispecific antibody molecule) or the antigen-binding fragment of the present invention.

In a fifth aspect, the present invention provides a vector comprising the nucleic acid encoding the anti-CD16A single-domain antibody, the monospecific antibody molecule or the antigen-binding fragment, or the multispecific antibody molecule or the antigen-binding fragment of the present invention, wherein preferably, the vector is an expression vector.

In a sixth aspect, the present invention provides a host cell comprising the isolated nucleic acid of the present invention or the vector of the present invention, wherein preferably, the host cell is prokaryotic or eukaryotic, more preferably, the host cell is selected from an *E. coli* cell, a yeast cell, a mammalian cell, and other cells suitable for preparing the antibody or the antigen-binding fragment, and most preferably, the host cell is a HEK293F cell.

In a seventh aspect, the present invention provides a method for preparing the anti-CD16A single-domain antibody, the monospecific antibody molecule or the antigen-binding fragment, or the multispecific antibody molecule or the antigen-binding fragment of the present invention, comprising culturing the host cell of the present invention under conditions suitable for expression of the nucleic acid encoding the anti-CD16A single-domain antibody, the monospecific antibody molecule, or the multispecific antibody molecule of the present invention, optionally recovering the anti-CD16A single-domain antibody, the monospecific antibody molecule, or the multispecific antibody molecule of the present invention from the host cell or a culture medium.

In an eighth aspect, the present invention provides a pharmaceutical composition comprising the anti-CD16A single-domain antibody, the monospecific antibody molecule or the antigen-binding fragment, or the multispecific antibody molecule or the antigen-binding fragment of the present invention, and a pharmaceutically acceptable carrier.

In some embodiments, the pharmaceutical composition of the present invention further comprises at least one additional active ingredient.

In a ninth aspect, the present invention provides use of the anti-CD16A single-domain antibody, the monospecific antibody molecule or the antigen-binding fragment, or the multispecific antibody molecule or the antigen-binding fragment of the present invention, and the pharmaceutical composition of the present invention, as a medicament for the treatment and/or prevention of a disease in an individual or as a diagnostic tool for a disease, wherein preferably, the individual is a mammal, and more preferably, the individual is a human.

In some embodiments, the anti-CD16A single-domain antibody, the monospecific antibody molecule or the antigen-binding fragment, the multispecific antibody molecule or the antigen-binding fragment of the present invention, and the pharmaceutical composition of the present invention are for use in the treatment and/or prevention or diagnosis of acute and chronic infectious diseases (e.g., bacterial or viral infections) or a tumor. For example, the tumor is selected from a solid tumor, a hematological cancer (e.g., leukemia, lymphoma, myeloma, e.g., multiple myeloma), and a metastatic lesion.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to the accurate arrangements and means of the embodiments shown in the drawings.
FIG. 1A shows binding of the anti-CD16A single-domain antibodies of the present invention to GS-CHO cells expressing human CD16A. MFI represents mean fluorescence intensity.
FIG. 1B shows no binding of the anti-CD16A single-domain antibodies of the present invention to GS-CHO cells expressing human CD16B.
FIG. 2A shows binding of the anti-CD16A single-domain antibodies of the present invention to human primary NK cells from a donor with ID No. SLB190084.
FIG. 2B shows binding of the anti-CD16A single-domain antibodies of the present invention to human primary NK cells from a donor with ID No. SLB190071.
FIG. 3A shows the structural forms of the bispecific antibodies of the present invention, wherein the left half of the bispecific antibodies in the column "Structural form" is an anti-CD16A single-domain antibody-Fc, the right half is an anti-TAA Fab-Fc, and the Fc regions have been subjected to a LALA modification and a knob-in-hole modification.
FIG. 3B shows the level of binding of anti-CD16A/HER2 bispecific antibodies to human primary NK cells.
FIG. 3C shows the level of binding of anti-CD16A/BCMA bispecific antibodies to human primary NK cells.
FIG. 4A shows killing of SK-BR3 cells (SK-BR3 cells belong to a breast cancer cell strain with high HER2 expression) by anti-CD16A/HER2 bispecific antibodies by ADCC. In the figure, "RLU" represents "relative luminescence unit", and PBS without the antibody is used as a blank control.
FIG. 4B shows no killing effect of anti-CD16A/HER2 bispecific antibodies on NCI-H929 cells (NCI-H929 cells belong to a multiple myeloma cell strain negative for HER2 expression).
FIG. 5A shows killing of NCI-H929 cells (NCI-H929 belongs to a multiple myeloma cell strain positive for BCMA expression) by anti-CD 16A/BCMA bispecific antibodies by ADCC.
FIG. 5B shows no killing effect of anti-CD16A/BCMA bispecific antibodies on SK-BR3 cells (SK-BR3 cells belong to a breast cancer cell strain negative for BCMA expression).
FIG. 6A shows activation of NK cells to target SK-BR3 cells (SK-BR3 cells belong to a breast cancer cell strain with high HER2 expression) by anti-CD16A/HER2 bispecific antibodies, wherein the ordinate indicates IFN-γ⁺ NK cells %.
FIG. 6B shows no activation of NK cells to target NCI-H929 cells (NCI-H929 cells belong to a multiple myeloma cell strain negative for HER2 expression) by anti-CD16A/HER2 bispecific antibodies, wherein the ordinate indicates IFN-γ⁺NK cells %.
FIG. 6C shows activation of NK cells to target SK-BR3 cells (SK-BR3 cells belong to a breast cancer cell strain with high HER2 expression) by anti-CD16A/HER2 bispecific antibodies, wherein the ordinate indicates CD107a⁺ NK cells %.
FIG. 6D shows no activation of NK cells to target NCI-H929 cells (NCI-H929 cells belong to a multiple myeloma cell strain negative for HER2 expression) by anti-CD16A/HER2 bispecific antibodies, wherein the ordinate indicates CD107a⁺ NK cells %.
FIG. 7A shows activation of NK cells to target NCI-H929 cells (NCI-H929 belongs to a multiple myeloma cell strain positive for BCMA expression) by anti-CD16A/BCMA bispecific antibodies, wherein the ordinate indicates IFN-γ⁺ NK cells %.
FIG. 7B shows no activation of NK cells to target SK-BR3 cells (SK-BR3 cells belong to a breast cancer cell strain negative for BCMA expression) by anti-CD16A/BCMA bispecific antibodies, wherein the ordinate indicates IFN-γ⁺ NK cells %.
FIG. 7C shows activation of NK cells to target NCI-H929 cells (NCI-H929 belongs to a multiple myeloma cell strain positive for BCMA expression) by anti-CD16A/BCMA bispecific antibodies, wherein the ordinate indicates CD107a⁺ NK cells %.
FIG. 7D shows no activation of NK cells to target SK-BR3 cells (SK-BR3 cells belong to a breast cancer cell strain negative for BCMA expression) by anti-CD16A/BCMA bispecific antibodies, wherein the ordinate indicates CD107a⁺ NK cells %.
FIG. 8A shows antigen-specific mediation of killing of a tumor cell strain positive for high HER2 expression by NK cells by anti-CD16A/HER2 bispecific antibodies.
FIG. 8B shows antigen-specific mediation of killing of a tumor cell strain positive for low HER2 expression by NK cells by anti-CD16A/HER2 bispecific antibodies.
FIG. 8C shows no activation of NK cells to target NCI-H929 cells (NCI-H929 cells belong to a multiple myeloma cell strain negative for HER2 expression) by anti-CD16A/HER2 bispecific antibodies.
FIG. 9A shows antigen-specific mediation of killing of a BCMA-positive tumor cell strain by NK cells by anti-CD16A/BCMA bispecific antibodies.
FIG. 9B shows no activation of NK cells to target a BCMA-negative tumor cell strain by anti-CD16A/BCMA bispecific antibodies.

### DETAILED DESCRIPTION

Before the present invention is described in detail, it should be understood that the present invention is not limited to the particular methods or experimental conditions described herein as the methods and conditions may vary. Additionally, the terms used herein are for the purpose of describing particular embodiments only and are not intended to be limiting.

### I. Definitions

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

The term "antibody" is used herein in the broadest sense, refers to a protein comprising an antigen-binding site, and encompasses natural and artificial antibodies with various structures, including but not limited to monoclonal antibodies, polyclonal antibodies, multispecific antibodies (e.g., bispecific antibodies), single-chain antibodies, intact antibodies, and antibody fragments. Preferably, the antibody of the present invention is a single-domain antibody or a heavy-chain antibody.

"Antibody fragment" or "antigen-binding fragment" refers to a molecule different from an intact antibody consisting of two heavy chains and two light chains, which comprises a portion of the intact antibody and binds to an antigen to which the intact antibody binds. Examples of the antibody fragment include, but are not limited to, an Fv, a Fab, a Fab', a Fab'-SH, an F(ab')₂; a diabody; a linear antibody; a single-chain antibody (e.g., scFv); a single-domain antibody; a bivalent or bispecific antibody or a fragment thereof; a camelid antibody (heavy-chain antibody); and a bispecific antibody or multispecific antibody formed from antibody fragments.

As used herein, the term "epitope" refers to a moiety of an antigen (e.g., human CD16A) that specifically interacts with an antibody molecule.

An antibody that shows identical or similar binding affinity and/or specificity as a reference antibody refers to an antibody that is capable of having at least 50%, 60%, 70%, 80%, 90%, or 95% or more of the binding affinity and/or specificity of the reference antibody. This can be determined by any method known in the art for determining binding affinity and/or specificity.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to epitopes. The CDRs of the heavy chain are generally referred to as CDR1, CDR2, and CDR3, and are numbered sequentially from the N-terminus. In a given amino acid sequence of a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (http://imgt.cines.fr/), and North CDR definition based on the affinity propagation clustering using a large number of crystal structures.

Unless otherwise stated, the term "CDR" or "CDR sequence" used herein encompasses CDR sequences determined by any one of the schemes above.

CDRs can also be determined based on having identical AbM numbering positions as a reference CDR sequence (e.g., any of the exemplary CDRs of the present invention). In one embodiment, the positions of CDRs of the single-domain antibody of the present invention are determined according to the AbM numbering scheme.

Unless otherwise stated, residue positions of antibody variable regions and CDRs (including heavy chain variable region residues) are numbered according to the AbM numbering scheme.

Antibodies with different specificities (i.e., different binding sites for different antigens) have different CDRs. However, although CDRs vary from antibody to antibody, only a limited number of amino acid positions within the CDRs are directly involved in antigen binding. The smallest overlapping region can be determined using at least two of the Kabat, Chothia, AbM, and Contact schemes, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the minimal binding unit may remain unchanged, while the remaining CDR residues defined according to Kabat or Chothia or AbM may be replaced by conservative amino acid residues.

The term "single-domain antibody" generally refers to an antibody in which a single variable domain (e.g., a heavy chain variable domain VHH derived from a camelid heavy-chain antibody) can impart antigen binding. That is, the single variable domain requires no interaction with another variable domain to recognize a target antigen. "Single-domain antibody" consists of only one variable region (also referred to as a variable domain) comprising, from the C-terminus to the N-terminus, FR4-CDR3-FR3-CDR2-FR2-CDR1-FR1, which may be produced naturally in camels or by genetic engineering techniques. A single-domain antibody is the minimal unit known to bind to a target antigen.

As used herein, the term "heavy-chain antibody (hcAb)" refers to an antibody without a light chain, which may comprise VH-CH2-CH3 or VH-CH1-CH2-CH3 from the N-terminus to the C-terminus, and may form a homodimer, such as a heavy-chain antibody dimer without light chains. The heavy-chain antibody of the present invention may comprise a VH from a standard antibody or a VH from a single-domain antibody. For example, the VH in the heavy-chain antibody of the present invention may be a single-domain antibody.

As used herein, the term "multispecific" antibody refers to an antibody having at least two antigen-binding sites, each of which binds to a different epitope of the same antigen or a different epitope of a different antigen. A multispecific antibody is an antibody having binding specificities for at least two different epitopes. In one embodiment, provided herein is a bispecific antibody having binding specificities for a first antigen and a second antigen.

The term "effector function" refers to biological activities attributed to an immunoglobulin Fc region that vary with immunoglobulin isotype. Examples of immunoglobulin effector functions include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down regulation of cell surface receptors (such as B-cell receptors), and B-cell activation.

"Human antibody" refers to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

The term "Fc region" is used herein to define a C-terminus region of an immunoglobulin heavy chain, which comprises at least a portion of a constant region. The term includes Fc regions of native sequences and variant Fc regions. In certain embodiments, a human IgG heavy chain Fc region extends from Cys226 or Pro230 to the carbonyl end of the heavy chain. However, the C-terminal lysine (Lys447) of the Fc region may or may not be present. Unless otherwise stated, the numbering of amino acid residues in the Fc region or constant region is based on the EU numbering scheme, which is also referred to as EU index, as described in Kabat et al., Sequences of Proteins of Immunological Interest, 5th Ed., Public Health Service, National Institutes of Health, Bethesda, MD, 1991.

The term "variable region" or "variable domain" refers to a domain of a heavy chain or light chain of an antibody involved in the binding of the antibody to an antigen. Variable domains of heavy chains and light chains of natural antibodies typically have similar structures, wherein each domain comprises four conserved framework regions (FRs) and three complementarity determining regions (CDRs). (See, e.g., Kindt et al., Kuby Immunology, 6th ed., W.H. Freeman and Co., p. 91 (2007)). A single VH or VL domain may be sufficient to provide antigen-binding specificity.

As used herein, the term "bind" (or "binding") or "specifically bind" (or "specific binding") means that the binding effect is selective for antigens and can be distinguished from unwanted or non-specific interactions. The ability of an antibody to bind to a particular antigen can be determined by an enzyme-linked immunosorbent assay (ELISA), SPR or biolayer interferometry or a conventional binding assay known in the art.

The term "effective amount" refers to an amount or dosage of the CD16A or composition of the present invention which generates expected effects in a patient in need of treatment or prevention after administration to the patient in a single dose or multiple doses. The effective amount can be easily determined by an attending physician as a person skilled in the art by considering a variety of factors as follows: species such as mammals; weight, age, and general health condition; the specific disease involved; the extent or severity of the disease; response in an individual patient; specific antibody administered; mode of administration; bioavailability characteristics of the administered preparation; selected administration regimen; and use of any concomitant therapy.

"Therapeutically effective amount" refers to an amount effective to achieve a desired therapeutic result at a necessary dose for a necessary period of time. The therapeutically effective amount of an antibody or antibody fragment, or a conjugate or composition thereof may vary depending on a variety of factors such as disease state, age, sex, and weight of an individual, and the ability of the antibody or antibody portion to elicit a desired response in an individual. The therapeutically effective amount is also such an amount that any toxic or adverse effect of the antibody or antibody fragment, or the conjugate or composition thereof is inferior to the therapeutically beneficial effect. "Therapeutically effective amount" preferably inhibits a measurable parameter (e.g., tumor growth rate and tumor volume) by at least about 20%, more preferably at least about 40%, even more preferably at least about 50%, 60%, or 70%, and still more preferably at least about 80% or 90%, relative to untreated subjects. The ability of a compound to inhibit a measurable parameter (e.g., cancer) can be evaluated in an animal model system that predicts efficacy in human tumors.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The terms "individual" and "subject" are used interchangeably and include mammals. The mammals include, but are not limited to, domesticated animals (e.g., cows, sheep, cats, dogs, and horses), primates (e.g., humans and non-human primates such as monkeys), rabbits and rodents (e.g., mice and rats). In particular, the individual or subject is a human.

The terms "tumor" and "cancer" are used interchangeably herein and encompass solid and liquid tumors.

The terms "cancer" and "carcinoma" refer to a physiological disease in mammals in which cell growth is unregulated.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein.

The term "cancer-associated antigen", "tumor-associated antigen (TAA)", or "tumor antigen" are used interchangeably to refer to molecules (generally proteins, carbohydrates, or lipids) preferably expressed completely or as fragments (e.g., MHC/peptide) on the surface of cancer cells, compared to normal cells, and the molecules can be used in the preferential targeting of cancer cells by the pharmaceutical agent. In some embodiments, the cancer-associated antigen is a cell surface molecule that has overexpression in tumor cells compared to normal cells, such as 1-fold overexpression, 2-fold overexpression, 3-fold overexpression, or more fold overexpression compared to normal cells. In some embodiments, the tumor-associated antigen is a cell surface molecule inappropriately synthesized in cancer cells, such as a molecule comprising deletions, additions, or mutations compared to molecules expressed on normal cells. The tumor-associated antigen (TAA) includes, but is not limited to, B cell maturation antigen (BCMA), prostate specific membrane antigen (PSMA); receptor tyrosine kinase-like orphan receptor 1 (ROR1); Fms-like tyrosine kinase 3 (FLT3); tumor-associated glycoprotein 72 (TAG72); carcinoembryonic antigen (CEA); epithelial cell adhesion molecule (EPCAM); mesothelin; interleukin 11 receptor α (IL-11Ra); prostate stem cell antigen (PSCA); protease serine 21 (Testisin or PRSS21); vascular endothelial growth factor receptor 2 (VEGFR2); human epidermal growth factor receptor 2 (Her2); cell surface associated mucin 1 (MUC1); bone marrow stromal cell antigen 2 (BST2); EGF module-containing mucin-like hormone receptor-like 2 (EMR2); and lymphocyte antigen 75 (LY75).

"Tumor immune escape" refers to a process by which tumors evade immune recognition and clearance. As such, as a therapeutic concept, tumor immunity is "treated" when such evasion diminishes, and the tumor is recognized and attacked by the immune system. Examples of tumor recognition include tumor binding, tumor shrinkage, and tumor clearance.

The term "infectious disease" refers to a disease caused by a pathogen, including, e.g., viral infection, bacterial infection, fungal infection, or caused by a protozoan, e.g., parasitic infection.

The term "chronic infection" refers to an infection in which an infectious agent (e.g., a pathogen such as a virus, bacteria, protozoa such as a parasite, fungus, or the like) has induced an immune response in an infected host, but has not been cleared or eliminated from the host as in acute infections. The chronic infection can be persistent, latent, or slow.

The term CD16A- "binding molecule" includes any antibody and antigen-binding fragment, and a fusion protein and conjugate thereof that selectively and specifically bind to CD16A, none of which specifically binds to CD16B. The CD16A binding molecule includes, but is not limited to, the specific anti-CD16A single-domain antibody, the monospecific antibody molecule, or the multispecific antibody molecule of the present invention.

"Isolated" CD16A-binding molecule refers to one that has been separated from components of its natural environment. In some embodiments, the CD16A-binding molecule is purified to a purity greater than 95% or 99% as determined by, e.g., electrophoresis (e.g., SDS-PAGE, isoelectric focusing (IEF) and capillary electrophoresis) or chromatography (e.g., ion exchange or reverse-phase HPLC). For a review of methods for assessing antibody purity, see, e.g., Flatman et al., J. Chromatogr., B848: 79-87 (2007).

"Isolated" nucleic acid refers to a nucleic acid molecule that has been separated from components of its natural environment. The isolated nucleic acid includes a nucleic acid molecule contained in a cell that typically comprises the nucleic acid molecule, but the nucleic acid molecule exists extrachromosomally or at a chromosomal location that is different from its natural chromosomal location. "Isolated nucleic acid encoding a CD16A-binding molecule" refers to one or more nucleic acid molecules encoding chains of a CD16A-binding molecule or a fragment thereof, including such nucleic acid molecules in a single vector or separate vectors, and such nucleic acid molecules present at one or more positions in a host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48: 444-453; available at http://www.gcg.com) which has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6.

A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) which has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

As used herein, the term "hybridization under conditions of low stringency, medium stringency, high stringency, or extreme stringency" describes hybridization and washing conditions. Instructions for performing hybridization reactions can be found in Current Protocols in Molecular Biology, John Wiley & Sons, N.Y. (1989), 6.3.1-6.3.6, which is incorporated by reference. Aqueous and non-aqueous methods are described in the references and either of the methods can be used. In some embodiments, the specific hybridization conditions mentioned herein are as follows: 1) the low stringency hybridization condition is in 6× sodium chloride/sodium citrate (SSC) at about 45 °C, followed by two washes in 0.2× SSC, 0.1% SDS at least at 50 °C (for the low stringency condition, the temperature of the washes can be increased to 55 °C); 2) the medium stringency hybridization condition is in 6× SSC at about 45 °C, followed by one or more washes in 0.2× SSC, 0.1% SDS at 60 °C; 3) the high stringency hybridization condition is in 6× SSC at about 45 °C, followed by one or more washes in 0.2× SSC, 0.1% SDS at 65 °C; and preferably, 4) the extreme stringency hybridization condition is in 0.5 M sodium phosphate, 7% SDS at 65 °C, followed by one or more washes in 0.2× SSC, 0.1% SDS at 65 °C. The extreme stringency condition (4) is a preferred condition and the one that should be used unless otherwise stated.

The term "pharmaceutical composition" refers to a composition that exists in a form allowing effective biological activity of the active ingredient contained therein, and does not contain additional ingredients having unacceptable toxicity to a subject to which the composition is administered.

As used herein, "treatment" (or "treat" or "treating") refers to slowing, interrupting, arresting, alleviating, stopping, lowering, or reversing the progression or severity of an existing symptom, disorder, condition, or disease. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present invention is used to delay the progression of a disease or to slow the progression of a disease.

As used herein, "prevention" (or "prevent" or "preventing") includes the inhibition of the development or progression of symptoms of a disease or disorder, or a specific disease or disorder. In some embodiments, subjects with family history of cancer are candidates for preventive regimens. Generally, in the context of cancer, the term "prevention" (or "prevent" or "preventing") refers to the administration of a drug prior to the onset of signs or symptoms of a cancer, particularly in subjects at risk of cancer.

The term "vector" as used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of nucleic acids to which they are operatively linked. Such vectors are referred to as "expression vectors" herein.

The term "host cell" refers to a cell into which an exogenous polynucleotide has been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein. Host cells are any type of cell systems that can be used to produce the antibody molecule of the present invention, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or a cultured plant tissue or animal tissue.

### II. CD16A-Binding Molecule of the Present Invention

Human CD16 (also referred to as FcγRIII) has two subtypes, CD16A (FcγRIIIA) and CD16B (FcyRIIIB), which share 96% amino acid sequence identity in their extracellular immunoglobulin-binding region sequences (van de Winkel and Capel, 1993, Immunol Today 14(5): 215-221).

CD16A is a transmembrane receptor expressed on macrophages, mastocytes, and NK cells. CD16B is present on polymorphonuclear granulocytes (PMNs) and is unable to trigger killing of tumor cells (van de Winkel and Capel, 1993, Immunol Today 14(5): 215-221). In addition, CD16B may be present as a soluble receptor in serum, and upon binding to antibodies *in vivo,* may cause side effects by forming immune complexes.

As a mediator of cellular innate immunity, NK cells are specialized cell killers and unlike T cells, tend to exist in a constitutively active state without the need for additional pre-stimulation. In contrast, resting cytotoxic T cells need to be activated by binding to MHC antigen complexes and subsequent co-stimulation by CD28. Therefore, among all immune effector cells, the NK cell is one of candidates of interest for immunotherapy.

In the prior art, it is unable to distinguish between the two subtypes CD16A and CD16B of most anti-CD16 antibodies. However, the anti-CD16 antibody of the present invention is capable of specifically binding to CD16A without binding to CD16B, and is capable of activating NK cells after binding to CD16A.

CD16A has several allelic polymorphisms, particularly at positions 48 and 158 of the IgG-binding region. The gene frequencies of the alleles CD16A158F and CD16A158V are about 0.6 and 0.4, respectively. The CD16A-binding molecule of the present invention is capable of recognizing these two alleles, CD16A158F and CD16A158V, thereby enabling the activation of the NK cell population by binding to CD16A in any patient.

In one embodiment, the CD16A-binding molecule of the present invention is an anti-CD16A single-domain antibody (sdAb), which comprises, from the N-terminus to the C-terminus, three complementarity determining regions, HCDR1, HCDR2, and HCDR3 (Kabat numbering) as follows:
(a) HCDR1 set forth in an amino acid sequence YNFTRVYMG (SEQ ID NO: 23) or a variant having no more than 3, no more than 2, or no more than 1 amino acid residue replacement of HCDR1 set forth in SEQ ID NO: 23, HCDR2 set forth in an amino acid sequence AISWGGGSTH (SEQ ID NO: 24) or a variant having no more than 3, no more than 2, or no more than 1 amino acid residue replacement of HCDR2 set forth in SEQ ID NO: 24, and HCDR3 set forth in an amino acid sequence ALDDYSGVLSP (SEQ ID NO: 25) or a variant having no more than 3, no more than 2, or no more than 1 amino acid residue replacement of HCDR3 set forth in SEQ ID NO: 25;
(b) HCDR1 set forth in an amino acid sequence FTFDMYVMT (SEQ ID NO: 26) or a variant having no more than 3, no more than 2, or no more than 1 amino acid residue replacement of HCDR1 set forth in SEQ ID NO: 26, HCDR2 set forth in an amino acid sequence AIHEDGGKTD (SEQ ID NO: 27) or a variant having no more than 3, no more than 2, or no more than 1 amino acid residue replacement of HCDR2 set forth in SEQ ID NO: 27, and HCDR3 set forth in an amino acid sequence VAYDYSYSEWYDY (SEQ ID NO: 28) or a variant having no more than 3, no more than 2, or no more than 1 amino acid residue replacement of HCDR3 set forth in SEQ ID NO: 28;
(c) HCDR1 set forth in an amino acid sequence FKFNSFVMT (SEQ ID NO: 29) or a variant having no more than 3, no more than 2, or no more than 1 amino acid residue replacement of HCDR1 set forth in SEQ ID NO: 29, HCDR2 set forth in an amino acid sequence AIHEDGGKTD (SEQ ID NO: 30) or a variant having no more than 3, no more than 2, or no more than 1 amino acid residue replacement of HCDR2 set forth in SEQ ID NO: 30, and HCDR3 set forth in an amino acid sequence AAVTHSLYDY (SEQ ID NO: 31) or a variant having no more than 3, no more than 2, or no more than 1 amino acid residue replacement of HCDR3 set forth in SEQ ID NO: 31; or
(d) HCDR1 set forth in an amino acid sequence FTFDWYWMG (SEQ ID NO: 32) or a variant having no more than 3, no more than 2, or no more than 1 amino acid residue replacement of HCDR1 set forth in SEQ ID NO: 32, HCDR2 set forth in an amino acid sequence AIHDDGTETH (SEQ ID NO: 33) or a variant having no more than 3, no more than 2, or no more than 1 amino acid residue replacement of HCDR2 set forth in SEQ ID NO: 33, and HCDR3 set forth in an amino acid sequence ATVRSDSYYDY (SEQ ID NO: 34) or a variant having no more than 3, no more than 2, or no more than 1 amino acid residue replacement of HCDR3 set forth in SEQ ID NO: 34.

The amino acid changes are additions, deletions, or substitutions of amino acids. For example, the amino acid changes are conservative amino acid substitutions.

In one embodiment, the anti-CD16A single-domain antibody of the present invention comprises:
(a) a heavy chain variable region comprising an amino acid sequence of SEQ ID NO: 1 or a sequence having at least 90%, 91%, 92%, 93%, 94%, 95%, 96%, 97%, 98%, or 99% identity thereto; 
EVQLVESGGGLVQPGGSLRLSCAASGYNFTRVYMGWFRQAPGKGREFVAAISWGGGSTHYADSVK
GRFTISRDNAKNTLYLQMNSLRAEDTAVYYCALDDYSGVLSPWGQGTLVTVSS (SEQ ID NO: 1)
(b) a heavy chain variable region comprising an amino acid sequence of SEQ ID NO: 2 or a sequence having at least 90%, 91%, 92%, 93%, 94%, 95%, 96%, 97%, 98%, or 99% identity thereto; 
QVQLVESGGGLVQPGGSLRLSCAASGFTFDMYVMTWFRQAPGKGREFVAAIHEDGGKTDYADSVK
GRFTISRDNAKNTLYLQMNSLRAEDTAVYYCVAYDYSYSEWYDYWGQGTLVTVSS (SEQ ID NO: 2)
(c) a heavy chain variable region comprising an amino acid sequence of SEQ ID NO: 3 or a sequence having at least 90%, 91%, 92%, 93%, 94%, 95%, 96%, 97%, 98%, or 99% identity thereto; or 
QVQLVESGGGLVQPGGSLRLSCAASGFKFNSFVMTWFRQAPGKGREFVAAIHEDGGKTDYADSVKG
RFTISRDNAKNTLYLQMNSLRAEDTAVYYCAAVTHSLYDYWGQGTLVTVSS (SEQ ID NO: 3)
(d) a heavy chain variable region comprising an amino acid sequence of SEQ ID NO: 4 or a sequence having at least 90%, 91%, 92%, 93%, 94%, 95%, 96%, 97%, 98%, or 99% identity thereto.

QVQLVESGGGLVQPGGSLRLSCAASGFTFDWYWMGWFRQAPGKGREFVAAIHDDGTETHYADSVK
GRFTISRDNAKNTLYLQMNSLRAEDTAVYYCATVRSDSYYDYWGQGTLVTVSS (SEQ ID NO: 4)

In one embodiment, the CD16A-binding molecule of the present invention is a monospecific antibody molecule, which comprises the anti-CD16A single-domain antibody of the present invention (also referred to herein as the "anti-CD16A single-domain antibody moiety") as one structural part thereof and an immunoglobulin Fc region as another structural part thereof. Optionally, the anti-CD16A single-domain antibody is linked to the immunoglobulin Fc region by an amino acid linker, e.g., by an amino acid linker of between 1 and 20 amino acids in length. In some embodiments, at least 90% of the amino acid linker is amino acids glycine and/or serine. In some embodiments, the Fc region is from an IgG, e.g., IgG1, IgG2, IgG3, or IgG4. In some embodiments, the Fc region is from IgG1. In some embodiments, the Fc region is from human IgG1.

In some embodiments of the present invention, the amino acid changes described herein include substitutions, insertions, or deletions of amino acids. Preferably, the amino acid changes described herein are amino acid substitutions, preferably conservative substitutions.

In a preferred embodiment, the amino acid changes described herein occur in regions other than CDRs (e.g., in FRs). More preferably, the amino acid changes described herein occur in regions other than the single-domain antibody moiety. In some embodiments, the substitutions are conservative substitutions. The "conservative substitution" refers to a substitution of an amino acid with another amino acid of the same class, for example, a substitution of an acidic amino acid with another acidic amino acid, a substitution of a basic amino acid with another basic amino acid, or a substitution of a neutral amino acid with another neutral amino acid. Exemplary substitutions are shown in Table A below:

**Table A**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In certain embodiments, the CD16A-binding molecule provided herein is modified to increase or decrease the degree of glycosylation thereof. Addition or deletion of glycosylation sites of the CD16A-binding molecule can be conveniently achieved by changing the amino acid sequence to create or remove one or more glycosylation sites. When the CD16A-binding molecule comprises an Fc region, the carbohydrate attached to the Fc region can be changed. In some applications, modifications that remove undesired glycosylation sites may be useful, for example, removing fucose modules to enhance antibody-dependent cell-mediated cytotoxicity (ADCC) (see Shield et al., (2002) JBC277:26733). In other applications, galactosidylation modifications can be made to regulate complement-dependent cytotoxicity (CDC). In certain embodiments, one or more amino acid modifications can be introduced into the Fc region of the CD16A-binding molecule provided herein, thereby producing an Fc region variant to enhance, for example, the effectiveness of the CD 16A-binding molecule of the present invention in the treatment of a cancer or an infectious disease.

In one embodiment, the CD16A-binding molecule of the present invention comprises modifications in an immunoglobulin Fc region thereof that reduce the binding affinity of the CD16A-binding molecule of the present invention for FcyRIIIA (CD16A) for reducing or eliminating effector functions caused by the immunoglobulin Fc region. In one embodiment, the modifications are in the Fc region of the immunoglobulin molecule, particularly in the CH2 region thereof. In one embodiment, the immunoglobulin molecule comprises an amino acid replacement at position 329 (EU numbering) of an immunoglobulin heavy chain. In one specific embodiment, the amino acid replacement is P329G. In one embodiment, the immunoglobulin molecule comprises amino acid replacements at positions 234 and 235 (EU numbering) of an immunoglobulin heavy chain. In one specific embodiment, the amino acid replacements are L234A and L235A (also referred to herein as "LALA mutation").

In one embodiment, the CD16A-binding molecule of the present invention comprises a protuberance ("knob") or a cavity ("hole") in each of two chains of the immunoglobulin Fc region thereof, and the protuberance or the cavity in the Fc region of one chain can be placed in the cavity or the protuberance in the Fc region of the other chain, such that the two chains form a stable "knob-in-hole" association with each other. In one embodiment, the amino acid replacement T366W is contained in the Fc region of one chain and the amino acid replacements T366S, L368A, and Y407V (EU numbering) are contained in the Fc region of the other chain. As such, the protuberance in one chain can be placed in the cavity in the other chain, which facilitates the correct pairing of the CD16A-binding molecule of the present invention.

In one specific embodiment, the immunoglobulin Fc region is an IgG1-Fc-LALA consensus heavy chain set forth in SEQ ID NO: 5.

DKTHTCPPCPAPEAAGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKSRWQQGN
VFSCSVMHEALHNHYTQKSLSLSPGK (SEQ ID NO: 5)

In one specific embodiment, one chain of the immunoglobulin Fc region has an amino acid sequence set forth in SEQ ID NO: 14.

DKTHTCPPCPAPEAAGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVYTLPPC
RDELTKNQVSLWCLVKGFYPSDIAVEWESNGQPENNYKTTPPVLDSDGSFFLYSKLTVDKSRWQQG
NVFSCSVMHEALHNHYTQKSLSLSPGK (IgG1-LALA-Fc-Knob, SEQ ID NO: 14)

In one specific embodiment, the other chain of the immunoglobulin Fc region has an amino acid sequence set forth in SEQ ID NO: 19.

ASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVSVLTVLHQDWLNGKEY
KCKVSNKALPAPIEKTISKAKGQPREPQVCTLPPSRDELTKNQVSLSCAVKGFYPSDIAVEWESNGQPE

NNYKTTPPVLDSDGSFFLVSKLTVDKSRWQQGNVFSCSVMHEALHNHYTQKSLSLSPGK
(IgG1-LALA-CH-Hole, SEQ ID NO: 19)

In one embodiment, the CD16A-binding molecule of the present invention, upon binding to CD16A expressed on a human cell, is capable of activating the cell or eliciting a series of biological responses, particularly signal responses. Preferably, the CD16A-binding molecule of the present invention, upon binding to a cell, is capable of triggering killing of the target cell in a manner analogous to antibody-dependent cell-mediated cytotoxicity (ADCC).

The present invention also provides a binding molecule that is specific for CD16A, non-specific for CD16B, and specific for one or more additional antigens. Therefore, in one embodiment, the CD16A-binding molecule of the present invention is a multispecific antibody molecule. The term "multispecific" herein means that the CD16A-binding molecule of the present invention, while lacking specificity for CD16B, has at least two specificities, one of which is for CD16A. The multispecific antibody molecule of the present invention includes, for example, bispecific, trispecific, and tetraspecific molecules. For example, the bispecific antibody includes a single-chain bispecific antibody, a two-chain bispecific antibody, a three-chain bispecific antibody, and the like.

The multispecific binding molecule of the present invention is capable of targeting macrophages or NK cells to an additional antigen, for example, to cells carrying or expressing the additional antigen, such that the macrophages or NK cells can destroy the cells carrying or expressing the additional antigen by phagocytosis or NK cell-mediated cell killing.

In some embodiments, the multispecific binding molecule of the present invention also has one or more specificities for a tumor-associated antigen (TAA), thereby enabling targeting of NK cells to tumor cells, wherein the multispecific binding molecule binds to CD16A, activates the NK cells, and produces killing of the tumor cells.

In some embodiments, the additional antigen is a cell surface antigen, e.g., CD19, CD20, CD30, laminin receptor precursor (also referred to as oncofetal antigen-immature laminin receptor (OFA-iLR)), EGFR1 (also referred to as HER-1 or ErbB1), EGFR2 (also referred to as HER-2, ErbB2, or neu), EGFR3 (also referred to as HER-3), Ep-CAM, PLAP (placental alkaline phosphatase), Thomsen-Friedenreich (TF) antigen, MUC-1 (mucin), IGFR, IL4-Rα, IL13-R, FcεRI, or CD5.

In a further embodiment, the additional antigen is an allergen, or a molecule associated with an allergic response in the body, such as (inter alia) IgE or FcεRI receptor on mastocytes.

Antibodies and antigen-binding fragments capable of specifically binding to CD19, CD20, CD30, laminin receptor precursor, EGFR1, EGFR2, EGFR3, Ep-CAM, PLAP, Thomsen-Friedenreich (TF) antigen, MUC-1 (mucin), IGFR, CD5, IL4-Rα, IL13-R, FcεRI, and IgE for use in the present invention are described in the prior art, see, e.g., EP-A-1314741; Reff et al., 1994 Blood 83: 435-445; EP-A-1005870; WO 01/92340; US 6,033,876; WO 86/03223; Buto et al., 1997 Int. J. Biol. Markers 12: 1-5; US 6,699,473; WO 98/50433; US 5,770195; EP-A-0502812; Carter et al., 1992 PNAS 89: 4285-4289; US 5,968,511; WO 04/106383; Nouri et al., 2000 BJU Int. 86: 894-900; EP-A-0429242; Ravn et al., 2004 J. Mol. Biol., 343: 985-996; Dahlenborg et al., 1997 Int. J. Cancer 70: 63-71; WO 88/05054; WO 02/053596; WO 04/071529; Studnicka et al., 1994 Protein Eng., 7: 805-814; Presta et al., 1993 J. Immunol., 151: 2623-2632). The sequences of antibodies and antibody fragments disclosed in these documents in the prior art can be used by those skilled in the art to prepare the binding moiety of the multispecific antibody molecule described herein that is directed against the additional antigen.

In some embodiments, the CD16A-binding molecule of the present invention is capable of binding to alleles CD16A 158F and CD16A 158V with similar affinity. For example, the binding molecule of the present invention binds to the two CD16A 158 alleles with no more than a 2-fold difference in affinity. In one preferred embodiment of the present invention, the specificity for CD16A is provided by the single-domain antibody of the invention.

The CD16A-binding molecule of the present invention may further comprise another functional domain. The functional domain may have the property of binding to a molecular effector. For example, the functional domain may be an FcR-binding peptide or an antibody Fc region capable of binding to an Fc receptor, with effector properties on the binding molecule. Alternatively, the functional domain may be an enzyme having the ability to convert a prodrug into an active pharmaceutical ingredient. Therefore, the CD16A-binding molecule of the present invention may be used in the antibody-directed enzyme prodrug therapy (ADEPT). In one specific embodiment, the functional domain of the binding molecule is a protein or peptide capable of increasing the serum half-life of the binding molecule, e.g., serum albumin or the Fc fragment of IgG, which can increase the serum half-life of the binding molecule by binding to FcyRn (neonatal Fc receptor). In one specific embodiment, the functional domain may also be a cytokine.

In some embodiments, the CD 16A-binding molecule of the present invention can bind to a labeling molecule or toxin. When the labeling molecule or toxin is a protein, it may be conjugated to the binding molecule by a peptide bond or by chemical coupling. The term "coupling" means that the two components are linked together by a chemical bond that includes a peptide bond, an ester bond, or a disulfide bond.

The binding of the binding molecule of the present invention to a marker molecule, such as a radioactive label, a fluorescent label, or a luminescent (including chemiluminescent) label, enables the binding molecule to be used as an immunostaining reagent. The immunostaining reagent can be used to detect tissue infiltrating NK cells, macrophages, or mastocytes that express CD16A, or can be used for the diagnosis of disease or the monitoring of disease progression or disease remission.

The binding molecule of the present invention can be conjugated to toxin molecules such as ribosyltransferase, serine protease, DNA alkylating agent, or mitotic inhibitor (e.g., doxorubicin) for targeting and killing NK cells and macrophages. Such binding molecules can be used as immunosuppressants. It will be understood by those skilled in the art that in this aspect of the present invention, the binding molecule is preferably specific for only one antigen, namely CD16A. Inhibition of the immune system is achieved using a binding molecule consisting of a monovalent antigen-binding fragment that selectively and specifically binds to CD16A. Such monovalent antigen-binding molecules, which are preferred as molecules blocking the CD16A receptor, are not cross-linked and therefore cannot activate NK cells or macrophages.

### III. Nucleic Acid of the Present Invention and Host Cell Comprising Same

In one aspect, the present invention provides a nucleic acid encoding any of the above CD16A-binding molecules or the fragments thereof or any one of the chains thereof. In one embodiment, provided is a vector comprising the nucleic acid. In one embodiment, the vector is an expression vector. In one embodiment, provided is a host cell comprising the nucleic acid or the vector. In one embodiment, the host cell is eukaryotic. In another embodiment, the host cell is selected from a yeast cell, a mammal cell (e.g., CHO cell or 293 cell), and other cells suitable for preparing an antibody or an antigen-binding fragment thereof. In another embodiment, the host cell is prokaryotic.

For example, the nucleic acid of the present invention includes a nucleic acid encoding an amino acid sequence selected from any one of SEQ ID NOs: 1-4, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 1-4.

The present invention further provides a nucleic acid hybridized under a stringency condition with the following nucleic acid or a nucleic acid encoding a polypeptide sequence having one or more amino acid substitutions (e.g., conservative substitutions), deletions or insertions compared with the following nucleic acid: a nucleic acid encoding an amino acid sequence selected from any one of SEQ ID NOs: 1-4; or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 1-4.

In one embodiment, provided are one or more vectors comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is a pcDNA3.1 vector.

Once the expression vector or DNA sequence has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, lipid-based transfection, or other conventional techniques. In the case of protoplast fusion, cells are cultured in a culture medium and screened for appropriate activity. Methods and conditions for culturing the resulting transfected cells and for recovering the resulting antibody molecules are known to those skilled in the art and may be changed or optimized according to the particular expression vector and the particular mammalian host cell used based on the present description and methods known in the prior art.

Additionally, cells having stably incorporated DNA in chromosomes thereof can be selected by introducing one or more markers permitting the selection of transfected host cells. The markers may, for example, provide prototrophy, biocidal (e.g., antibiotics) resistance, or heavy metal (e.g., copper) resistance, etc., for an auxotrophic host. Selectable marker genes may be connected directly to a DNA sequence to be expressed or introduced through co-transformation into the same cell. Additional elements may also be required for optimal synthesis of mRNA. The elements may include splicing signals, transcriptional promoters, enhancers, and termination signals.

In one embodiment, provided is a host cell comprising the polynucleotide of the present invention. In some embodiments, provided is a host cell comprising the expression vector of the present invention. In some embodiments, the host cell is selected from a yeast cell, a mammalian cell, and other cells suitable for preparing an antibody. Suitable host cells include prokaryotic microorganisms, such as E. *coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include monkey kidney CV1 line (COS-7) transformed by SV40; human embryonic kidney line (HEK 293 or 293F cells), 293 cell, baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (Hep G2), Chinese hamster ovary cell (CHO cell), CHOS cell, NSO cell, and myeloma cell line such as Y0, NS0, P3X63, and Sp2/0. For reviews of mammalian host cell lines suitable for protein production, see, e.g., Yazaki and Wu, Methods in Molecular Biology, vol. 248 (B. K. C. Lo ed., Humana Press, Totowa, NJ), pp. 255-268 (2003). In one preferred embodiment, the host cell is a CHO cell or a 293 cell.

### IV. Production and Purification of the CD16A-Binding Molecule of the Present Invention

In one embodiment, the present invention provides a method for preparing a CD16A-binding molecule, which comprises culturing a host cell comprising a nucleic acid encoding the CD16A-binding molecule or an expression vector comprising the nucleic acid under conditions suitable for expression of the nucleic acid encoding the CD16A-binding molecule, and optionally isolating the CD16A-binding molecule. In a certain embodiment, the method further comprises recovering the CD16A-binding molecule from the host cell (or host cell culture medium).

For recombinant production of the CD16A-binding molecule of the present invention, a nucleic acid encoding the CD16A-binding molecule of the present invention is first isolated, and inserted into a vector for further cloning and/or expression in a host cell. Such nucleic acids can be easily isolated and sequenced by using conventional procedures, for example, by using an oligonucleotide probe that is capable of specifically binding to the nucleic acid encoding the CD16A-binding molecule of the present invention.

The CD16A-binding molecule of the present invention prepared as described herein can be purified by known prior art such as high performance liquid chromatography, ion exchange chromatography, gel electrophoresis, affinity chromatography, and size exclusion chromatography. The actual conditions used to purify a particular protein also depend on factors such as net charge, hydrophobicity and hydrophilicity, and these will be apparent to those skilled in the art. The purity of the CD16A-binding molecule of the present invention can be determined by any one of a variety of well-known analytical methods including size exclusion chromatography, gel electrophoresis, high performance liquid chromatography, and the like.

### V. Activity Assay on the CD 16A-Binding Molecule of the Present Invention

The CD16A-binding molecule provided herein can be identified, screened, or characterized for physical/chemical properties and/or biological activities through a variety of assays known in the art. In one aspect, the CD16A-binding molecule of the present invention is tested for the antigen-binding activity, for example, by known methods such as ELISA and Western blotting. The binding to CD16A can be determined by methods known in the art, and exemplary methods are disclosed herein. In some embodiments, the binding of the CD16A-binding molecule of the present invention to CD16A is determined by SPR or biolayer interferometry.

The present invention also provides an assay for identifying a CD16A-binding molecule having biological activity. The biological activity may include, for example, binding to CD16A on the surface of a cell (e.g., NK cell), cytotoxic effects, and the like.

Cells used in any of the above *in vitro* assays include a cell line that naturally expresses CD16A or is engineered to express CD16A. The cell line that is engineered to express CD16A is a cell line that does not normally express CD16A and expresses CD16A after transfection of DNA encoding CD16A into the cells.

### VI. Pharmaceutical Composition and Pharmaceutical Preparation

In some embodiments, the present invention provides a composition comprising any of the CD16A-binding molecules or the immunoconjugates thereof described herein, wherein preferably, the composition is a pharmaceutical composition. In one embodiment, the composition further comprises a pharmaceutically acceptable carrier. In one embodiment, the composition (e.g., the pharmaceutical composition) comprises the CD16A-binding molecule or the immunoconjugate thereof of the present invention, and a combination of one or more additional therapeutic agents (e.g., chemotherapeutic agents, cytotoxic agents, additional antibodies, anti-infective active agents, small molecule drugs, or immunomodulatory agents, e.g., anti-PD-1 antibody or anri-PD-L1 antibody).

In some embodiments, the composition is used for preventing or treating a tumor. In some embodiments, the tumor is a cancer.

The present invention further includes a composition (including a pharmaceutical composition or a pharmaceutical preparation) comprising a CD16A-binding molecule or an immunoconjugate thereof, and/or a composition (including a pharmaceutical composition or a pharmaceutical preparation) comprising a polynucleotide encoding the CD16A-binding molecule. These compositions may further comprise a suitable pharmaceutically acceptable carrier, such as a pharmaceutical excipient known in the art, including a buffer.

As used herein, the "pharmaceutically acceptable carrier" includes any and all solvents, dispersion media, isotonic agents, absorption delaying agents, and the like, which are physiologically compatible. The pharmaceutical carrier suitable for use in the present invention can be sterile liquid, such as water and oil, including those of petroleum, animal, plant, or synthetic origin, such as peanut oil, soybean oil, mineral oil, or sesame oil. Water is a preferred carrier when the pharmaceutical composition is administered intravenously. Saline solutions, aqueous dextrose, and glycerol solutions can also be used as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. For use and application of the excipients, see Handbook of Pharmaceutical Excipients, 5th edition, R. C. Rowe, P. J. Seskey and S. C. Owen, Pharmaceutical Press, London, Chicago. The composition may further comprise a small amount of wetting agent, emulsifier, or pH buffer, if desired. The compositions may be in the form of a solution, a suspension, an emulsion, a tablet, a pill, a capsule, a powder, a sustained release preparation, or the like. Oral preparations may comprise a standard pharmaceutical carrier and/or excipient such as pharmaceutical-grade mannitol, lactose, starch, magnesium stearate and saccharin.

The pharmaceutical preparation, preferably in the form of a lyophilized preparation or an aqueous solution, comprising the CD16A-binding molecule described herein can be prepared by mixing the CD16A-binding molecule of the present invention of desired purity with one or more optional pharmaceutically acceptable carriers *(*Remington's Pharmaceutical Sciences, 16th edition, Osol, A. ed. (1980)).

The pharmaceutical composition or preparation of the present invention may further comprise more than one active ingredient required by a particular indication treated, preferably those having complementarity activities without adversely affecting one another. For example, it may be desirable to further provide additional anti-cancer or anti-infective active ingredients, such as chemotherapeutic agents, cytotoxic agents, additional antibodies, anti-infective active agents, small molecule drugs, or immunomodulatory agents, e.g., anti-PD-1 antibody or anti-PD-L1 antibody. The active ingredients are suitably combined in an amount effective for an intended purpose.

A sustained release preparation can be prepared. Suitable examples of the sustained release preparation include a semipermeable matrix of a solid hydrophobic polymer comprising the CD16A-binding molecule of the present invention. The matrix is in the form of a shaped article, e.g., a film or a microcapsule.

### VII. Combination Product or Kit

In some embodiments, the present invention also provides a combination product comprising the CD16A-binding molecule or the antibody fragment thereof of the present invention, or the immunoconjugate thereof, and one or more additional therapeutic agents (e.g., chemotherapeutic agents, additional antibodies, cytotoxic agents, anti-infective active agents, small molecule drugs or immunomodulatory agents). In some embodiments, the additional antibody is, e.g., an anti-PD-1 antibody or an anti-PD-L1 antibody.

In some embodiments, the combination product is used for preventing or treating a tumor. In some embodiments, the tumor is a cancer or the like.

In some embodiments, two or more ingredients in the combination product may be administered to a subject in combination sequentially, separately, or simultaneously.

In some embodiments, the present invention also provides a kit comprising the CD16A-binding molecule, the pharmaceutical composition, the immunoconjugate, or the combination product of the present invention, and optionally a package insert directing administration.

In some embodiments, the present invention also provides a pharmaceutical article of manufacture comprising the CD16A-binding molecule, the pharmaceutical composition, the immunoconjugate, or the combination product of the present invention, and optionally a package insert directing administration.

### VIII. Use of the CD16A-Binding Molecule of the Present Invention

In one embodiment, the present invention relates to a method for preventing or treating a tumor (e.g., cancer) in a subject, which comprises administering to the subject an effective amount of the CD16A-binding molecule or the pharmaceutical composition, the immunoconjugate, or the combination product comprising same disclosed herein. In some embodiments, the tumor is a tumor subjected to immune escape.

In one embodiment, the present invention relates to a method for preventing or treating an infectious disease in a subject, which comprises administering to the subject an effective amount of the CD16A-binding molecule or the pharmaceutical composition, the immunoconjugate, or the combination product comprising same disclosed herein.

In another aspect, the present invention relates to a method for inducing NK cell-mediated cytotoxicity in a subject, which comprises administering to the subject an effective amount of the CD16A-binding molecule or the pharmaceutical composition, the immunoconjugate, or the combination product comprising same disclosed herein.

The subject may be a mammal, e.g., a primate, preferably a higher primate, e.g., a human (e.g., a patient suffering from or at risk of suffering from the disease described herein). In one embodiment, the subject suffers from or is at risk of suffering from the disease described herein (e.g., the tumor or infectious disease as described herein). In certain embodiments, the subject is receiving or has received additional therapies, e.g., chemotherapy and/or radiotherapy. Alternatively or in combination, the subject is immunocompromised due to infection or is at risk of being immunocompromised due to infection.

In some embodiments, the tumor, e.g., cancer, described herein includes, but is not limited to, a solid tumor, a hematological cancer, a soft tissue tumor, and a metastatic lesion.

Examples of the solid tumor include a malignant tumor, e.g., sarcomas and carcinomas (including adenocarcinomas and squamous cell carcinomas) of various organ systems, such as carcinomas that affect the liver, lungs, breasts, lymph, gastrointestinal tract (e.g., the colon), genitourinary tract (e.g., the kidney and bladder epithelial cells), prostate, and pharynx. The adenocarcinomas include malignant tumors such as most colon cancers, rectal cancers, renal cell carcinomas, liver cancers, non-small cell lung cancers in lung cancers, small intestine cancers, and esophageal cancers. The squamous cell carcinomas include malignant tumors, such as carcinomas in the lungs, esophagus, skin, head and neck regions, oral cavity, anus, and cervix. In one embodiment, the cancer is melanoma, e.g., advanced melanoma. In one embodiment, the cancer is renal cell carcinoma. Metastatic lesions of the aforementioned carcinomas can also be treated or prevented by using the method and composition of the present invention.

Non-limiting examples of preferred cancers for treatment include lymphoma (e.g., diffuse large B-cell lymphoma, Hodgkin's lymphoma, non-Hodgkin's lymphoma), breast cancer (e.g., metastatic breast cancer), liver cancer (e.g., hepatocellular carcinoma (HCC)), lung cancer (e.g., non-small cell lung cancer (NSCLC), e.g., stage IV or recurrent non-small cell lung cancer, NSCLC adenocarcinoma, or NSCLC squamous cell carcinoma), myeloma (e.g., multiple myeloma), leukemia (e.g., chronic myelogenous leukemia), skin cancer (e.g., melanoma (e.g., stage III or IV melanoma), or Merkel cell carcinoma), head and neck cancer (e.g., head and neck squamous cell carcinoma (HNSCC)), myelodysplastic syndrome, bladder cancer (e.g., transitional cell carcinoma), kidney cancer (e.g., renal cell carcinoma, e.g., clear cell renal cell carcinoma, e.g., advanced or metastatic clear cell renal cell carcinoma), and colon cancer. In addition, refractory or recurrent malignant tumors can be treated using the CD16A-binding molecule or the pharmaceutical composition, the immunoconjugate, or the combination product comprising same described herein.

In some embodiments, the CD16A-binding molecule or the immunoconjugate, the composition, or the combination product comprising same of the present invention delays the onset of a disorder and/or symptoms associated with the disorder.

In some embodiments, the method for prevention or treatment described herein further comprises administering to a subject or individual the CD16A-binding molecule, the pharmaceutical composition, the immunoconjugate, or the combination product disclosed herein, and one or more additional therapies, e.g., therapeutic modalities and/or additional therapeutic agents.

In some embodiments, the therapeutic modality includes surgery (e.g., tumor resection), radiotherapy (e.g., external beam therapy that involves three-dimensional conformal radiotherapy in which an irradiation region is designed), partial irradiation (e.g., irradiation directed to a preselected target or organ), focused irradiation, and the like. The focused irradiation may be selected from stereotactic radiosurgery, fractionated stereotactic radiosurgery, and intensity-modulated radiotherapy. The focused irradiation may have a radiation source selected from particle beams (protons), cobalt-60 (photons), and linear accelerators (X-rays), for example, as described in WO 2012/177624.

The radiotherapy can be performed through one or a combination of methods including, but not limited to, external beam therapy, internal radiotherapy, implant irradiation, stereotactic radiosurgery, systemic radiotherapy, radiotherapy and permanent or transient interstitial brachytherapy.

In some embodiments, the therapeutic agent is selected from a chemotherapeutic agent, a cytotoxic agent, an additional antibody, an anti-infective active agent, a small molecule drug, and an immunomodulatory agent (e.g., an activator of a co-stimulatory molecule or an inhibitor of an immune checkpoint molecule).

The combination therapy of the present invention encompasses both co-administration (wherein two or more therapeutic agents are contained in the same preparation or separate preparations) and separate administration. In the case of separate administration, administration of the CD16A-binding molecule, the immunoconjugate, or the like, of the present invention can be performed prior to, concurrently with, and/or subsequent to administration of additional therapies.

In one embodiment, administration of the CD16A-binding molecule and administration of additional therapies (such as therapeutic modalities or therapeutic agents) occur within about one month, or within about one, two, or three weeks, or within about 1, 2, 3, 4, 5, or 6 days from each other.

The CD16A-binding molecule of the present invention (and the pharmaceutical composition or the immunoconjugate comprising same) can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal, or subcutaneous administration. The administration may be performed by any suitable route, such as injection, e.g., intravenous or subcutaneous injection, to some extent depending on short-term or long-term treatment. Various administration schedules are encompassed herein, including but not limited to, single administration or multiple administrations at multiple time points, bolus injection, and pulse infusion.

In order to prevent or treat a disease, the appropriate dosage of the CD16A-binding molecule of the present invention (when used alone or in combination with one or more additional therapeutic agents) will depend on the type of a disease to be treated, the type of a CD16A-binding molecule, severity and progression of the disease, the purpose of administration (prophylactic or therapeutic) of the CD16A-binding molecule, previous treatments, clinical history of a patient, response to the CD16A-binding molecule, and the discretion of an attending physician. The CD16A-binding molecule is suitably administered to a patient as a single treatment or through a series of treatments. The dosage and treatment regimen of the CD16A-binding molecule can be determined by those skilled.

It will be understood that any of the above prevention or treatments can be performed using the immunoconjugate, the composition, or the combination of the present invention in place of the CD16A-binding molecule.

### IX. Methods and Compositions for Diagnosis and Detection

In certain embodiments, any of the CD16A-binding molecules provided herein can be used to detect the presence of CD16A in a biological sample. The term "detection" as used herein includes quantitative and qualitative detections, and exemplary detection methods may involve immunohistochemistry, immunocytochemistry, flow cytometry (e.g., FACS), magnetic beads complexed with antibody molecules, and ELISA. In certain embodiments, the biological sample is blood, serum, or other liquid samples of biological origin. In certain embodiments, the biological sample includes cells or tissues. In some embodiments, the biological sample is derived from a hyperproliferative or cancerous lesion.

In one embodiment, provided is a CD16A-binding molecule for use in a diagnostic or detection method. In another aspect, provided is a method for detecting the presence of CD16A in a biological sample. In certain embodiments, the method comprises detecting the presence of the CD16A protein in a biological sample. In certain embodiments, the CD16A is human CD16A. In certain embodiments, the methods comprise contacting a biological sample with the CD16A-binding molecule as described herein under conditions that allow binding of the CD16A-binding molecule to CD16A, and detecting the formation of a complex between the CD16A-binding molecule and CD16A. The formation of the complex indicates the presence of CD16A. The method may be an *in vitro* or *in vivo* method. In one embodiment, the CD16A-binding molecule is used to select a subject suitable for treatment with the CD16A-binding molecule, e.g., wherein CD16A is a biomarker for selecting the subject.

It can be understood that the embodiments described in each part of the present invention, such as diseases, therapeutic agents, therapeutic modalities and administration, are also applicable to, or may be combined with, embodiments of other parts of the present invention. The embodiments described in each part of the present invention that are applicable to the properties, use and method of the CD16A-binding molecule are also applicable to the compositions, conjugates, combination products and kits comprising the CD16A-binding molecule.

The following examples are described to assist in understanding the present invention. The examples are not intended to be and should not be interpreted in any way as limiting the protection scope of the present invention.

### Examples

The present invention as generally described herein will be more easily understood by reference to the following examples, which are provided by way of illustration and are not intended to limit the scope of the present invention. These examples are not intended to indicate that the following experiments are all or only experiments performed.

### Example 1. Screening of Synthetic Libraries of Anti-CD16A Single-Domain Antibodies and Differential Staining by Flow Cytometry

By this example, four fully synthetic human single-domain antibodies targeting CD16A were obtained by screening. The specific procedures were as follows.

Synthetic libraries of single-domain antibodies after rearrangement of human germline genes were constructed (see C. F. Barbas III et al., eds. (2001), Phage display: a laboratory manual. Cold spring harbor laboratory press: Cold Spring Harbor, New York), and the single-domain antibodies were displayed on the surface of yeast cells.

The first round of screening was performed by magnetic-activated cell sorting using the MACS system from Miltenyi. Specifically, 2×10⁹ yeast cells were taken from each library, and after a biotinylated CD16A-158V protein (Aero Biosystems, CDA-H82E9) was added as an antigen, the cells were incubated at room temperature for 30 min. The cells were washed once with 50 mL of FACS buffer (1× PBS, containing 1% bovine serum albumin). After resuspension in 10 mL of FACS buffer, 40 µL of streptavidin microbeads (Miltenyi LS) were added, and the cells were incubated at 4 °C for 15 min. After incubation, the cell suspension was centrifuged at 3000 rpm for 3 min, the supernatant was discarded, and the cells were resuspended in 10 mL of FACS buffer.

The resulting cell suspension was loaded on a Miltenyi LS column. After loading, the Miltenyi LS column was washed 3 times with 3 mL of FACS buffer. The Miltenyi LS column was removed from the magnetic field and the yeast cells were eluted with 5 mL of growth medium YPD (Sangon Biotech, A507022-0250). The eluted yeast cells were collected, placed in a culture flask, cultured at 30 °C overnight, and then induced by shaking at 20 °C for 24 h.

The yeast library cells after the magnetic bead enrichment were subjected to a second round of sorting by flow cytometry. 4×10⁷ yeast cells were taken from the library and washed three times with a FACS buffer. After a FACS buffer containing a biotinylated CD16A-158V protein and a rabbit anti-Flag antibody (Sigma-Aldrich, F7425) was added, the cells were incubated at room temperature for 30 min. After the cells were washed twice with a FACS buffer, a FACS buffer containing streptavidin SA-PE (eBioscience, 12-4317-87) (1:200 dilution) and Alex Fluor-647 conjugated goat anti-mouse IgG (H + L) cross-adsorbing secondary antibody (ThermoFisher, A21235) (1:200 dilution) was added and incubated at 4 °C for 15 min in the dark. After the cells were washed twice with a pre-cooled FACS washing buffer, cell sorting was performed using the MoFlo_XDP ultra-rapid flow cytometry sorting system (Beckman Coulter, ML99030), and the sorted yeast cells were cultured overnight at 30 °C with shaking.

Subsequent screenings were performed using a biotinylated CD16A-158F protein (Aero Biosystems, CDA-H82E8), a biotinylated CD16B-NA1 protein (Aero Biosystems, CDB-H82E4), a biotinylated CD16B-NA2 protein (Aero Biosystems, CDB-H82Ea), and a biotinylated cyno CD16 protein (Aero Biosystems, FC6-C82E0) as antigens, respectively, by the method as described above for the second round of flow cytometry sorting.

After sorting, yeast monoclonals were selected and cultured in a 48-well plate at 30 °C overnight. Yeast plasmids were extracted for sequencing and the yeast cells were subjected to differential staining by flow cytometry. 1× 10⁶ cells were taken from each yeast clone and stained by the method as described above for the second round of flow cytometry sorting. Finally, single-domain antibody molecules that bind to CD16A-158V, CD16A-158F, and cyno CD16, and do not bind to CD16B-NA1 and CD16B-NA2 were obtained, and constructed into plasmid vectors expressed by eukaryotic cells for identification.

Through the above multiple rounds of screening, four clones binding to CD16A, i.e., clone Y88A3, clone Y95H1, clone Y99F6, and clone Y100D6, were obtained, and the expressed human single-domain antibodies were named Y88A3 sdAb (SEQ ID NO: 1), Y95H1 sdAb (SEQ ID NO: 2), Y99F6 sdAb (SEQ ID NO: 3), and Y100D6 sdAb (SEQ ID NO: 4), respectively, according to the clone numbers.

### Example 2. Expression and Purification of Anti-CD16A Heavy-Chain Antibodies in Eukaryotic Cells HEK293F

The nucleotide sequences of 4 exemplary anti-CD16A single-domain antibodies (Y88A3 sdAb, Y95H1 sdAb, Y99F6 sdAb, and Y100D6 sdAb) obtained by screening from the yeast display synthetic library of Example 1 were each constructed into a pcDNA3.1 plasmid vector (Invitrogen, wherein an IgG1-Fc-LALA consensus heavy chain sequence is set forth in SEQ ID NO: 5) containing an IgG1 heavy chain constant region with L234A and L235A mutations, and the resulting corresponding plasmid was extracted.

HEK293F cells were cultured to a density of 3.0× 10⁶ cells/mL. The resulting corresponding plasmid and the transfection reagent PEI were mixed homogeneously according to a mass ratio of 1:3, left to stand for 20 min, and then slowly added to the HEK293F cells, which were then cultured in a shaking incubator at 37 °C with 8% CO₂ for 6-7 days.

The cells were centrifuged, and the cell supernatant was collected and combined with the Protein A purification resin (GE Healthcare) at room temperature for 1 h. The Protein A purification resin was washed with phosphate buffered saline (PBS) at pH 7.0 to remove impure proteins, and then eluted with 0.1 M citric acid at pH 3.0. The eluted protein was exchanged into PBS by ultrafiltration to obtain an anti-CD16A heavy-chain antibody consisting of two heavy chains.

After the production of the obtained anti-CD16A heavy-chain antibody was determined, a small amount of the sample was subjected to size-exclusion chromatography (SEC) to determine whether aggregation of multiple heavy-chain antibodies occurred for the heavy chain antibody protein in the sample. The results showed that the anti-CD16A heavy-chain antibodies containing two sdAb-Fc-LALA chains from the N-terminus to the C-terminus purified by the above procedures all could have purity above 90%. The sample was subpackaged and then stored in a refrigerator at -80 °C.

### Example 3. Assay on Binding Affinity of Anti-CD16A Heavy-Chain Antibodies for Human CD16A or CD16B and Cynomolgus Monkey CD16A

The equilibrium dissociation constant (K_{D}) for binding of the purified 4 exemplary anti-CD16A heavy-chain antibodies obtained in Example 2 to human CD16A-158V, CD16A-158F, CD16B-NA1 and CD16B-NA2, and cynomolgus monkey CD16A (cyno-CD16A) antigens was determined by biolayer interferometry (BLI). In Example 2, to exclude the possible effect of the Fc fragment on binding to CD16, IgG 1- F c into which L234A and L235A mutations had been introduced, i.e., IgG1-Fc-LALA, was used as the Fc fragment to remove the binding of the Fc fragment to CD 16, thereby minimizing background interference.

An affinity assay was performed according to the method known in the art (Estep P., et al., High throughput solution based measurement of antibody-antigen affinity and epitope binning, mAbs, 2013, 5(2), 270-278). Briefly, the sensor was equilibrated offline in an assay buffer for 30 min, and was equilibrated online for 60 s to establish a baseline. The purified heavy-chain antibodies obtained as described above were each loaded online onto a Protein A sensor (ForteBio, 18-5010) for the ForteBio affinity assay. Then, the sensors loaded with the anti-CD16A heavy-chain antibodies were sequentially exposed to antigenic protein solutions of human CD16A-158V protein (ACRO Biosystems, CD9-H52H4), CD16A-158F protein (ACRO Biosystems, CDA-H5220), CD16B-NA1 protein (ACRO Biosystems, CDB-H5227) and CD16B-NA2 protein (ACRO Biosystems, CDB-H5222), and cyno-CD16A protein (ACRO Biosystems, FC6-C52H9) at a concentration of 100 nM for 100 s, before the sensors were transferred to an assay buffer for dissociation for 120 s to measure the dissociation rate. The fitting and analysis of binding kinetics were performed using a 1:1 binding model, and K_{D} values were calculated.

As shown in Table 1, the results showed that the 4 exemplary anti-CD16A heavy-chain antibodies all showed relatively high affinity for human CD16A-158V and CD16A-158F, which was not affected by the CD16A antigen subtype (158V or 158F), and was comparable to that of the CD16A humanized antibody 4LS21 (SEQ ID NO: 6 & NO: 7, sequence from PCT publication No. WO2006125668A2) from Affimed Therapeutics AG and significantly higher than that (about 100 nM) of the control antibody IgG1-Fc and CD16A-158V. Meanwhile, the 4 exemplary anti-CD16A heavy-chain antibodies described above all did not bind to human CD16B-NA1 and CD16B-NA2, bound to CD16 in the same manner as the control CD16A humanized antibody 4LS21, and had selectivity for binding to human CD16A, wherein the antibody 4LS21 was in the form of ScFv-Fc.

In addition, the 4 exemplary anti-CD16A heavy-chain antibodies all bound to cynomolgus monkey CD16A, indicating that the anti-human CD16A heavy-chain antibodies cross-bound to monkey CD16A.

**Table 1. Affinity of anti-CD16A heavy chain antibodies for binding to human CD16A or CD16B and monkey CD 16A**

| Antibody name | Affinity (K_{D}, M) | | | | |
|---|---|---|---|---|---|
| | hCD16A-158V | hCD16A-158F | hCD16B-NA1 | hCD16B-NA2 | Cyno-CD16A |
| 4LS21 antibody | 7.75×10⁻⁹ | 4.75×10⁻⁹ | N.B | N.B | 7.94×10⁻⁹ |
| Y88A3 heavy-chain antibody | 1.83×10⁻⁸ | 2.23×10⁻⁸ | N.B | N.B | 1.83×10⁻⁸ |
| Y95H1 heavy-chain antibody | 2.47×10⁻⁸ | 1.49×10⁻⁸ | N.B | N.B | 1.97×10⁻⁸ |
| Y99F6 heavy-chain antibody | 3.15×10⁻⁸ | 3.01×10⁻⁸ | N.B | N.B | 2.60×10⁻⁸ |
| Y100D6 heavy-chain antibody | 1.58×10⁻⁸ | 2.15×10⁻⁸ | N.B | N.B | 2.50×10⁻⁸ |

| | | | | | |
|---|---|---|---|---|---|
| Note: N.B is short for no binding. | | | | | |

### Example 4. Determination of Epitope of Human CD16A Bound by Anti-CD16A Heavy-Chain Antibodies

The amino acid sequences of human CD16A and CD16B mainly differ in sites at positions 129 and 140 of the N-terminus (Ellwanger K et al., Redirected optimized cell killing (ROCK®): A highly versatile multispecific fit-for-purpose antibody platform for engaging innate immunity. MAbs, 2019, 11(5), 899-918). Therefore, in this example, the site-directed mutagenesis of amino acids and gene cloning were used to mutate position G129 in the antigen human CD16A-158V to D129 of CD16B (i.e., G129D) or mutate position Y140 in the antigen human CD16A-158V to A140 (i.e., Y140A) to prepare a mutant antigen CD16A-158V-G129D (SEQ ID NO: 8) or CD16A-158V-Y140A (SEQ ID NO: 9) of human CD16A-158V. A 6× His tag was added to the C-terminus of the sequence of each mutant antigen to facilitate the purification. The gene sequences for the above two mutant antigens were each constructed into a pcDNA3.1 vector (purchased from Invitrogen, the nucleotide sequence of pcDNA3.1 vector is set forth in SEQ ID NO: 10), expressed by the expression method in Example 2, and purified by using Ni-NTA.

After the antigens were purified, the K_{D} values for the binding of the 4 exemplary anti-CD16A heavy-chain antibodies of the present invention to human CD16A-158V-G129D and CD16A-158V-Y140A were determined by the ForteBio method, so that the epitopes bound by the single-domain fragments of the anti-CD16A heavy-chain antibodies were determined. The 4LS21 antibody in the form of ScFv-Fc was used as a control.

The ForteBio affinity assay was performed by the method described in Example 3, using human CD16A-158V-G129D and CD16A-158V-Y140A as antigens. As shown in Table 2, the results showed that the Y88A3 single-domain antibody fragment, the Y99F6 single-domain antibody fragment, the Y100D6 single-domain antibody fragment, and the Y95H1 single-domain antibody fragment of the 4 exemplary anti-CD16A heavy-chain antibodies all showed no binding to the antigen CD16A-158V-Y140A but normal binding to CD16A-158V-G129D, indicating that Y140, but not G129, was critical for the selective binding of the Y88A3 single-domain fragment, the Y99F6 single-domain fragment, the Y100D6 single-domain fragment, and the Y95H1 single-domain fragment of the anti-CD16A heavy-chain antibodies to CD16A but not to CD16B, and Y140 was an important amino acid residue in the epitope. This was the same as for 4LS21 ScFv.

**Table 2. Binding affinity of anti-CD16A heavy-chain antibodies to mutated human CD16A**

| Antibody name | Affinity (K_{D}, M) | |
|---|---|---|
| | CD16A-158V-G129D | CD16A-158V-Y140A |
| 4LS21 antibody | 4.63×10⁻⁹ | N.B |
| Y88A3 heavy-chain antibody | 1.34×10⁻⁸ | N.B |
| Y95H1 heavy-chain antibody | 5.85×10⁻⁸ | N.B |
| Y99F6 heavy-chain antibody | 7.81×10⁻⁹ | N.B |
| Y100D6 heavy-chain antibody | 2.57×10⁻⁸ | N.B |

| | | |
|---|---|---|
| Note: N.B is short for no binding. | | |

### Example 5. Binding of Anti-CD16A Heavy-Chain Antibodies to GS-CHO Cells Expressing Human CD16A and CD16B

In this example, the binding of anti-CD16A heavy-chain antibodies (subjected to gradient dilution) to stable GS-CHO cell strains overexpressing human CD16A (type 158V) and CD16B (type NA1) on their surfaces was assayed by flow cytometry.

Specifically, cDNAs encoding human CD16A (type 158V) and CD16B (type NA1) were each cloned into a pXC17.4 plasmid vector (Lonza, SEQ ID NO: 11), which was then transfected into GS-CHO cells (Lonza) by electrotransfection. Human CD16A (type 158V) and CD16B (type NA1) were expressed on the cell membrane. By screening, GS-CHO cell strains stably expressing human CD16A (type 158V) and CD16B (type NA1) were obtained.

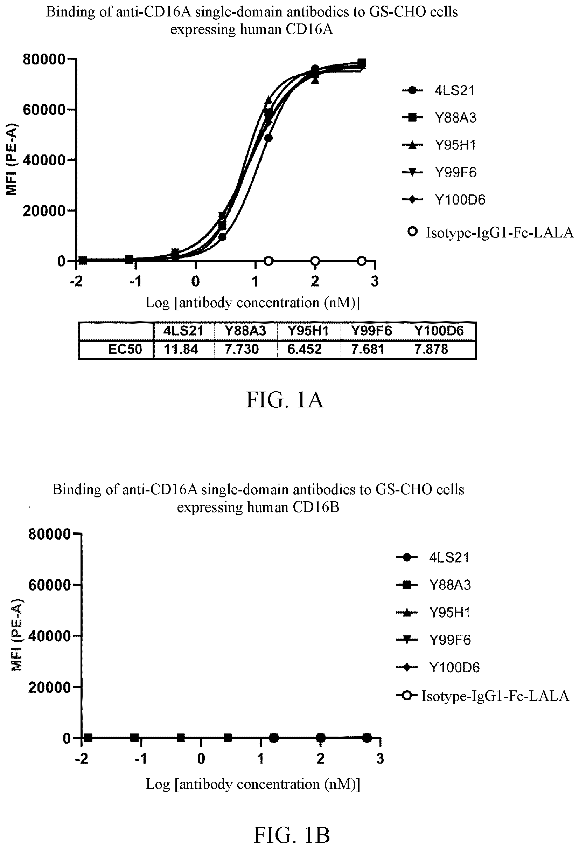

The procedures for the flow cytometry assay were as follows. The above GS-CHO cells expressing human CD16A (type 158V) and CD16B (type NA1) were centrifuged at 400 g for 5 min, and then the medium was removed. The cells were resuspended in PBS. After counting, the cells were adjusted to a density of 2×10⁶ cells/mL, and the cell suspension was added to a U-bottom 96-well plate at 100 µL/well. Then, the test anti-CD16A heavy-chain antibody was added to make a final concentration of 100 nM, and subjected to a 6-fold dilution to obtain 10 gradients. The cells and the sample were left to stand on ice for a reaction for 1 h. The mixture was centrifuged at 300 g for 5 min, and the supernatant was removed. The cells were washed once with PBS. The mixture was centrifuged at 300 g for 5 min, and PBS was removed. 100 µL of PE-anti-human Fc antibody (Southern Biotech, 2040-09) diluted at a ratio of 1:200 was added to each well. The mixture was incubated on ice for 30 min in the dark. The mixture was centrifuged at 400 g for 5 min, and the supernatant was removed. The cells were washed twice with PBS, and then resuspended in 100 µL of PBS. A fluorescence shift assay was performed by using a flow cytometer (BD, Facscelesta). The assay results are shown in FIGs. 1A and 1B.

As can be seen from FIGs. 1A and 1B, the Y88A3, Y99F6, Y95H1, and Y100D6 single-domain moieties of the anti-CD16A heavy-chain antibodies all had relatively strong binding to a GS-CHO stable cell strain overexpressing CD16A, with the half maximal effective concentration (EC₅₀) for binding being superior to that of the control antibody 4LS21; meanwhile, the Y88A3, Y99F6, Y95H1, and Y100D6 single-domain moieties of the anti-CD16A heavy-chain antibodies all did not bind to a GS-CHO stable cell strain overexpressing CD16B (type NA1), so that the anti-CD16A heavy-chain antibodies had the selectivity for CD16A. Isotype IgG1-Fc-LALA used as a negative control in FIGs. 1A and 1B was an anti-Hen Egg Lysozyme (HEL) antibody, with variable region sequences set forth in SEQ ID NO: 12 & SEQ ID NO: 13.

### Example 6. Binding of Anti-CD16A Heavy-Chain Antibodies to Primary Natural Killer (NK) Cells

In this example, the binding of anti-CD16A heavy-chain antibodies (subjected to gradient dilution) to primary NK cells was assayed by flow cytometry. The experimental procedures were as follows.

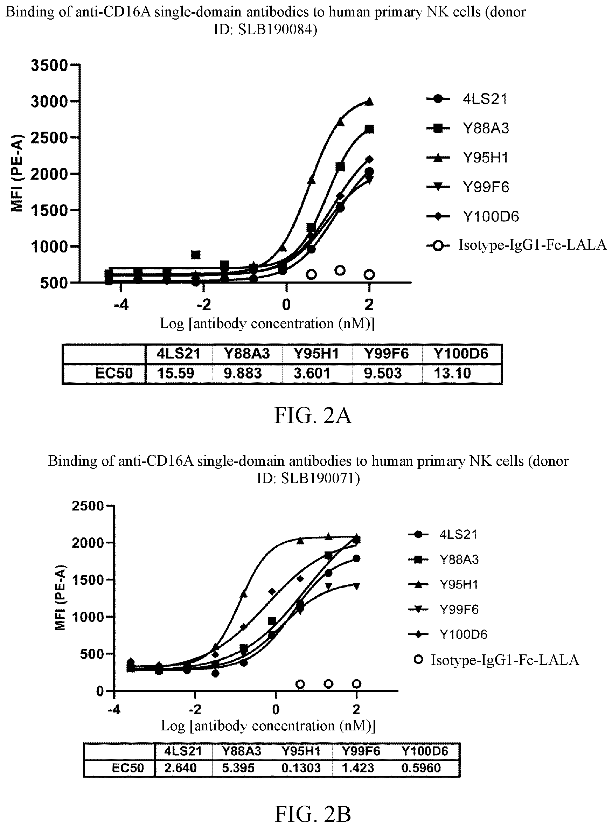

Human PBMC cells were thawed (Sailybio, SLB190084 & SLB190071), and NK cells were enriched and isolated using a human NK cell enrichment kit (Stemcell, 19055) and centrifuged at 400 g for 5 min. The supernatant was removed, and the cells were resuspended in PBS. After counting, the cells were adjusted to a density of 2×10⁶ cells/mL, and the cell suspension was added to a U-bottom 96-well plate at 100 µL/well. Then, the test antibody was added to make a final concentration of 100 nM, and subjected to a 6-fold dilution to obtain 10 gradients. The mixture was left to stand on ice for a reaction for 1 h. The mixture was centrifuged at 300 g for 5 min, and the supernatant was removed. The cells were washed once with PBS. The mixture was centrifuged at 300 g for 5 min, and PBS was removed. 100 µL of PE-anti-human Fc antibody (Southern Biotech, 2040-09) diluted at a ratio of 1:200 was added to each well. The mixture was incubated on ice for 30 min in the dark. The mixture was centrifuged at 400 g for 5 min, and the supernatant was removed. The cells were washed twice with PBS, and then resuspended in 100 µL of PBS. A fluorescence shift assay was performed by using a flow cytometer (BD, Facscelesta). The assay results are shown in FIGs. 2A and 2B.

As can be seen from FIGs. 2A and 2B, the Y88A3, Y99F6, Y95H1 and Y100D6 single-domain moieties of the anti-CD16A heavy-chain antibodies were all capable of binding to primary NK cells, with the binding activity comparable to or better than that of 4LS21. The control antibody used in FIGs. 2A and 2B was the same as that in Example 5.

### Example 7. Preparation of Anti-CD16A/HER2 Bispecific Antibodies and Anti-CD16A/BCMA Bispecific Antibodies

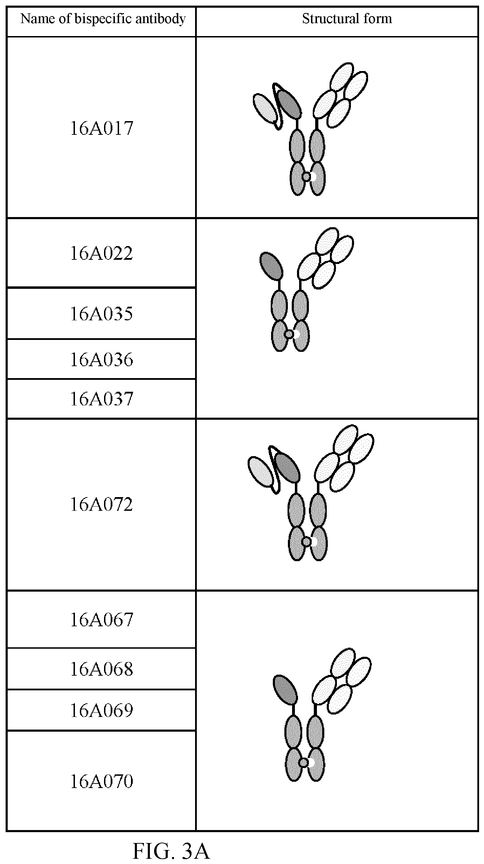

In order to verify whether the CD16A single-domain antibodies obtained in the present application have the ability to mediate NK cell activation and killing, in this example, the CD16A single-domain antibodies were combined with each of an anti-HER2 antibody (clone: 4D5) and an anti-BCMA antibody (clone: ADI384-56) for the construction of bispecific antibodies in the form of "1 + 1" (the numbers and the structural forms of the antibodies are shown in Table 3 and FIG. 3A), in which the heavy chain dimerization was performed by a Knob-into-Hole technique. The specific construction method was as follows: the sequences of the 4 exemplary anti-CD16A single-domain antibodies (Y88A3, Y95H1, Y99F6, and Y100D6) were each constructed into a pcDNA3.1-SP-IgG1 LALA-Fc-Knob vector (pcDNA3.1 was purchased from Invitrogen, and the LALA mutation and Knob mutation were performed by the PCR site-directed mutagenesis method, SEQ ID NO: 14); heavy chain variable regions (VHs) of the anti-HER2 antibody (clone ID: 4D5, SEQ ID NO: 15 & NO: 16) and the anti-BCMA antibody (clone ID: ADI384-56, SEQ ID NO: 17 & NO: 18) were each constructed into a pcDNA3.1-SP-IgG1 LALA-CH-Hole vector (pcDNA3.1 was purchased from Invitrogen, the LALA mutation and Hole mutation were performed by the PCR site-directed mutagenesis method, and the sequence of the IgG1-LALA-CH-Hole was set forth in SEQ ID NO: 19), while light chain variable regions (VLs) of the two antibodies were each constructed into a pcDNA3.1-SP-Igκ CL vector (purchased from Invitrogen, the amino acid sequence of Igκ CL was set forth in SEQ ID NO: 20) of the light chain constant region of IgG1. Corresponding plasmids were extracted according to the kit instructions.

HEK293F cells were cultured to a density of 3.0×10⁶ cells/mL. A plasmid for encoding a Knob heavy chain, a plasmid for encoding a Hole heavy chain, and a plasmid for encoding a light chain were mixed according to a ratio of 1:1:1, and the obtained plasmid mixture was mixed homogeneously with a transfection reagent PEI according to a volume ratio of 1:3. The mixture was left to stand for 20 min (the structural forms of the bispecific antibodies are shown in Table 3 and FIG. 3A) and then slowly added to HEK293F cells, which were subjected to expression and purification according to the method in Example 2. The SEC method was used to determine whether aggregation occurred in the bispecific antibody samples. The SEC results showed that the purified bispecific antibodies all could have purity above 90%.

**Table 3. Construction of anti-CD 16A/HER2 and anti-CD16A/BCMA bispecific antibodies**

| Name of bispecific antibody | Name of CD16A antibody clone | Name of anti-TAA and clone thereof |
|---|---|---|
| 16A017 | 4LS21 | Anti-HER2 (clone: 4D5) |
| 16A022 | Y88A3 | |
| 16A035 | Y99F6 | |
| 16A036 | Y100D6 | |
| 16A037 | Y95H1 | |
| 16A072 | 4LS21 | Anti-BCMA (clone: ADI384-56) |
| 16A067 | Y88A3 | |
| 16A068 | Y99F6 | |
| 16A069 | Y100D6 | |
| 16A070 | Y95H1 | |

| | | |
|---|---|---|
| Note: anti-TAA refers to an antibody for an anti-tumor-associated antigen, and in this example, TAA refers to each of HER2 and BCMA. | | |

### Example 8. Binding of Anti-CD16A/HER2 and Anti-CD16A/BCMA Bispecific Antibodies to NK Cells

In this example, the binding of anti-CD16A/HER2 and anti-CD16A/BCMA bispecific antibodies (subjected to gradient dilution) of the present invention to primary NK cells was assayed by flow cytometry. The assay method was the same as that in Example 6.

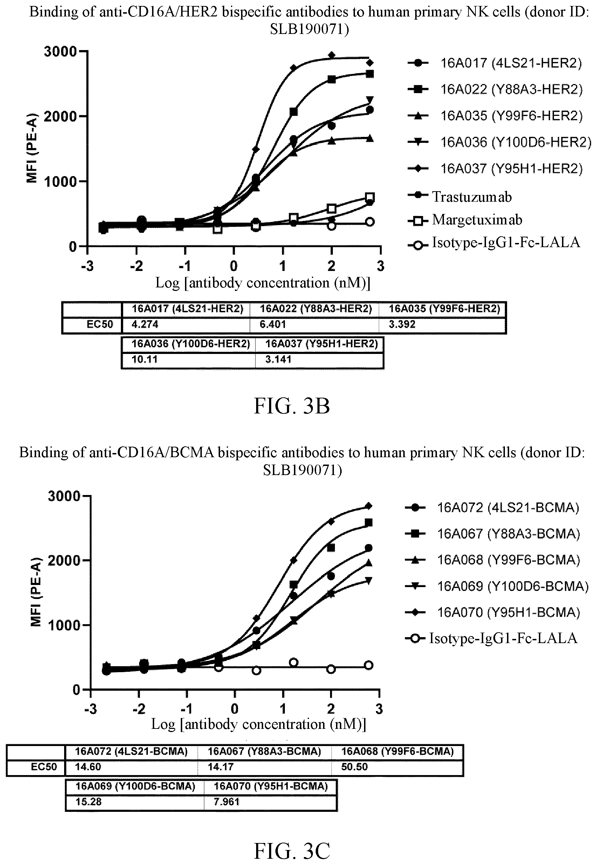

As shown in FIGs. 3B and 3C, the assay results showed that the anti-CD16A/HER2 and anti-CD16A/BCMA bispecific antibodies of the present invention all had the ability to bind to primary NK cells, which was comparable to that of the control 4LS21/anti-TAA bispecific antibody.

### Example 9. Preliminary Validation of Anti-CD16A/TAA Bispecific Antibodies Specifically Mediating Antibody-Dependent Cell-Mediated Cytotoxicity (ADCC) by Binding to CD16A and Tumor Antigen

In this example, Jurkat T cells expressing a CD16A NFAT luciferase reporter gene were used as effector cells (ADCC Jurkat-NFAT luc reporter) to determine the ability of the anti-CD16A/TAA bispecific antibodies to specifically mediate ADCC.

The anti-CD16A/TAA bispecific antibodies were each co-incubated with ADCC Jurkat-NFAT luc report cells and tumor cells (SK-BR3 cells, which belong to a breast cancer cell strain with high HER2 expression and negative BCMA expression and are obtained from ATCC; and NCI-H929 cells, which belong to a multiple myeloma cell strain with negative HER2 expression and positive BCMA expression and are obtained from ATCC), and the fluorescence signal values released by the ADCC Jurkat-NFAT luc report cells after incubation were determined, so that the ability of the anti-CD16A/TAA bispecific antibodies to specifically mediate effector cell signal activation and ADCC by binding to CD16A and a tumor antigen was reflected. The detailed experimental procedures were as follows.

ADCC Jurkat-NFAT luc reporter cells (Promega, G7102) were thawed in an RPMI-1640 complete medium (Gibco, 111835-030) containing 10% inactivated fetal bovine serum (HyClone, SH30406.05), 100 µg/mL hygromycin B (Gibco, 10687-010), 250 µg/mL antibiotic G-418 sulfate solution (Promega, V8091), 1 mM sodium pyruvate (Gibco, 11360), and 0.1 mM MEM NEAA (Gibco, 1114), and passaged according to the 48-hour passaging strategy at a passaging density of 2×10⁵ cells/mL. Before the experiment, ADCC Jurkat-NFAT luc reporter cells were washed three times with an RPMI-1640 assay medium containing 3% low IgG fetal bovine serum, and then resuspended to a density of 2.0× 10⁶ cells/mL for later use. SK-BR3 (HER2⁺, BCMA⁻) and NCI-H929 (HER2⁻, BCMA⁺) tumor cells were each digested with pre-warmed 0.25% trypsin-EDTA, and the solution obtained by the digestion was centrifuged at 400 g for 5 min. The cells were collected and counted, resuspended in the above assay medium, and adjusted to a cell concentration of 0.8×10⁶ cells/mL for later use. Each of the anti-CD16A/TAA bispecific antibodies was diluted with the above assay medium to make a final concentration of 100 nM, and then subjected to a 5-fold dilution to obtain 10 gradients. The following components were added to each well of the experimental group: 50 µL of ADCC Jurkat-NFAT luc reporter cells, 50 µL of antibody sample solution, and 50 µL of tumor cells. The mixture was mixed homogeneously by using a multichannel pipettor, centrifuged at 200 g for 3 min, and then mixed homogeneously. The cell culture plate was incubated in an incubator at 37 °C with 5% CO₂ overnight, and then 100 µL of the prepared Bio-Glo^{™} Luciferase Assay System (Promega, G7940) was added to each well. The plate was left to stand for 5-10 min in the dark, and then read for fluorescence signal values using a Max i3 microplate reader (Molecular Devices) with full wavelength scanning.

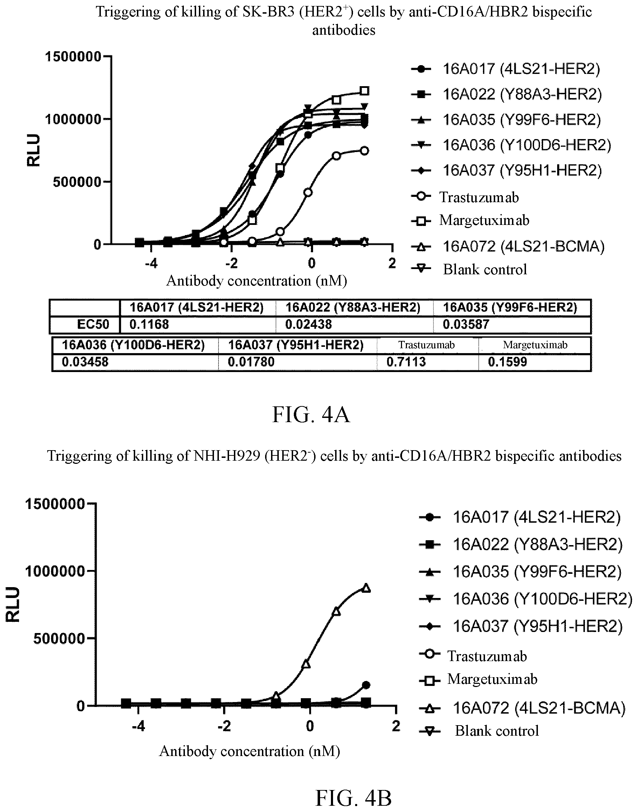

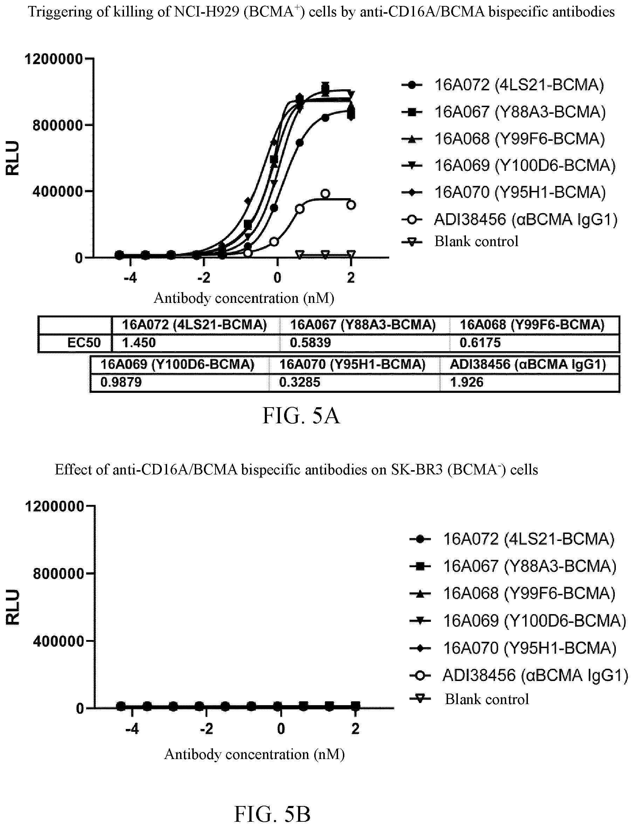

As shown in FIGs. 4A, 4B, 5A, and 5B, the experimental results showed that the anti-CD16A single-domain antibodies contained in the bispecific antibodies of the present invention could perform tumor-specific activation on ADCC Jurkat-NFAT luc reporter cells to generate an ADCC effect, had better cell activation and ADCC ability than the control antibody 4LS21/anti-TAA, and were superior to ADCC-enhanced IgG1 antibodies such as margetuximab (also referred to as MGAH22) (from MacroGenics, SEQ ID NO: 21 & NO: 22).

### Example 10. Anti-CD 16A/TAA Bispecific Antibodies Specifically Mediating NK Cell Activation by Binding to CD16A and Tumor Antigen

In this example, an intracellular flow cytometry staining method was used to determine the ability of the anti-CD16A/TAA bispecific antibodies to specifically mediate NK cell activation by binding to CD16A and a tumor antigen. The anti-CD16A/TAA bispecific antibodies were each co-incubated with NK cells and tumor cells, and the secretion of IFNγ in NK cells and the expression level of CD107a on the cell membrane surface after the activation were determined, so that the tumor antigen-specific activation effect of the anti-CD16A single-domain antibody moieties of the anti-CD16A/TAA bispecific antibodies on NK cells was reflected. The detailed experimental procedures were as follows.

PBMC cells were thawed (Sailybio, SLB190071), resuspended in an RPMI-1640 complete medium (Gibco, 111835-030) containing 200 µL/10 mL DNase I Solution (Stemcell, 07900) and 10% inactivated fetal bovine serum (HyClone, SH30406.05), and incubated at 37 °C for 2 h. NK cells were enriched according to the method provided by an NK enrichment kit (StemCell, 19055) from StemCell Technologies, and finally adjusted to a concentration of 2×10⁶ cells/mL. SK-BR3 (HER2⁺, BCMA⁻) and NCI-H929 (BCMA⁺, HER2⁻) tumor cells were each digested with pre-warmed 0.25% trypsin-EDTA, and the solution obtained by the digestion was centrifuged at 400 g for 5 min. The cells were collected and counted, resuspended in an RPMI-1640 complete medium containing AF488-conjugated anti-human CD107a (BioLegend, 328630) at a final concentration of 2 µg/mL, and adjusted to a concentration of 4×10⁵/mL. Each of the anti-CD16A/TAA bispecific antibodies was diluted with an RPMI-1640 complete medium to make a final concentration of 100 nM, and then subjected to a 6-fold dilution to obtain 10 gradients. A mixed sample solution of PMA (Sigma-Aldrich, P8139) at a final concentration of 100 ng/mL and ionomycin (Cell Signaling, 9995S) at a final concentration of 1 µg/mL was prepared in an RPMI-1640 complete medium and used as a positive control. An appropriate amount of mixed solution of BFA (BD, 347688) at a final concentration of 10 µg/mL and BD GolgiStop^{™} (BD, 51-2092KZ) at a final concentration of 10 µg/mL was prepared in an RPMI-1640 complete medium to block the transfer of cytokines to the outside of cells so as to determine the accumulation of the cytokines in the cells.

The following components were added to each well of the experimental group: 50 µL of NK cells, 50 µL of BD GolgiStop^{™}/BFA solution, 50 µL of antibody sample or PMA/ionomycin solution, and 50 µL of tumor cell suspension. The mixture was mixed homogeneously by using a multichannel pipettor, centrifuged at 200 g for 5 min, and then mixed homogeneously. The cell culture plate was incubated in an incubator at 37 °C with 5% CO₂ for 6 h. The cells were centrifuged at 400 g for 5 min, and then the supernatant was removed. 200 µL of BD Cytofix^{™} Fixation buffer (BD, 554655) was added to each well, and the cells were fixed at 4 °C for 20 min in the dark. After fixation, the cells were centrifuged at 400 g for 5 min, and then the supernatant was discarded. The cells were resuspended in BD FACS Perm Buffer III (BD, 558050), stained at 4 °C for 40 min in the dark, and then washed twice with BD Perm/Wash^{™} Buffer (BD, 51-2091KZ) at 200 µL/well. The cells were stained with a BD Perm/Wash^{™} buffer containing 1 µg/mL PE-conjugated anti-lFN-γ (BioLegend, 506507), APC-conjugated anti-CD56 (Invitrogen, 17-0567-41), and BV421-conjugated anti-CD3 (BioLegend, 300434) at 4 °C for 1 h in the dark, washed, and then assayed on a flow cytometer (BD, FACSCELESTA). After CD3⁻/CD56⁺ cells were delineated, the NK cells were determined for the expression levels of CD107a and lFN-γ.

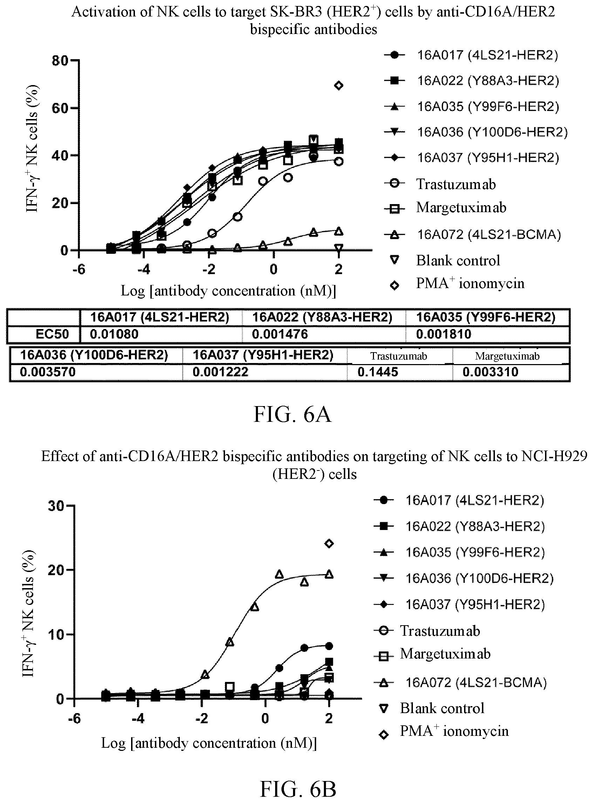

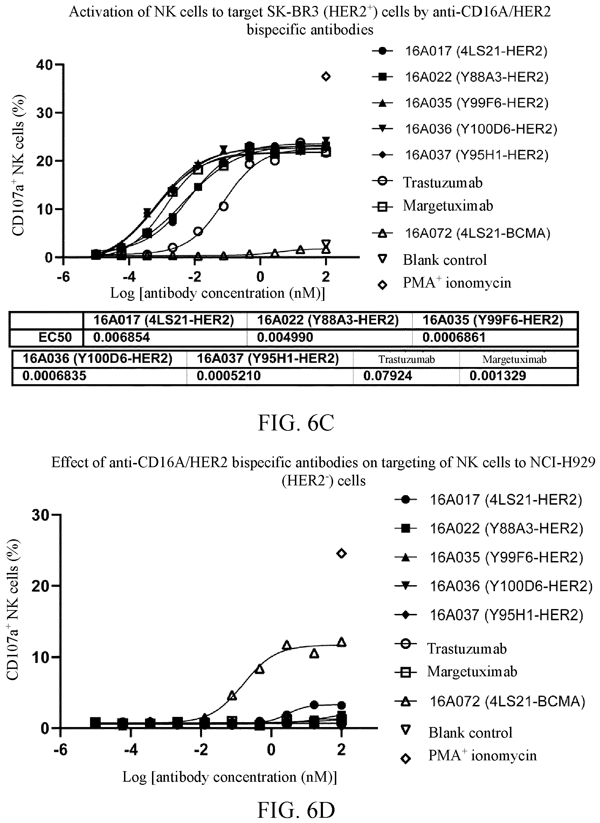

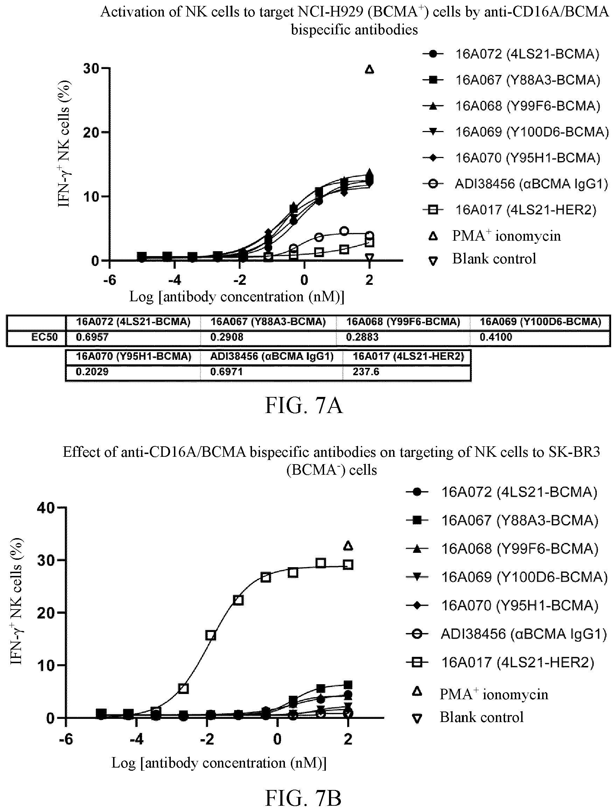

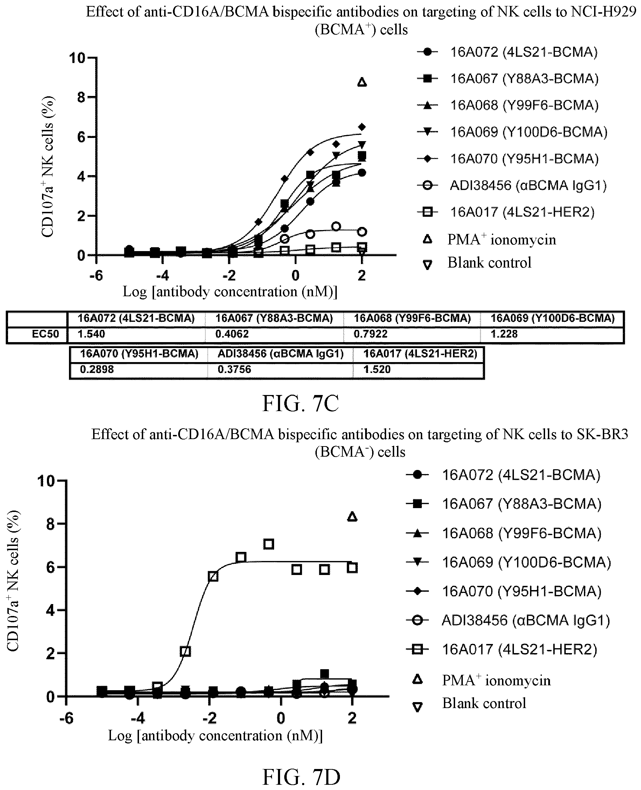

As shown in FIGs. 6A-6D and FIGs. 7A-7D, the experimental results showed that the anti-CD16A/HER2 and anti-CD16A/BCMA bispecific antibodies of the present invention could perform tumor-specific upregulation on the expression levels of CD107a and lFN-γ of NK cells, had a comparable or better NK cell activation ability as compared to the 4LS21/anti-TAA bispecific antibody as a control, and were superior to ADCC-enhanced IgG1 antibodies such as margetuximab.

### Example 11. Anti-CD 16A/TAA Bispecific Antibodies Specifically Mediating Killing of Target Cells by NK Cells

In this study, the anti-CD16A/TAA bispecific antibodies were each co-incubated with NK cells and tumor cells, and the level of lactate dehydrogenase (LDH) in the system was determined, so that the ability of the anti-CD16A/TAA bispecific antibodies to mediate antigen-specific killing of tumor cells by NK cells was reflected. The detailed experimental procedures were as follows.

Human PBMC cells were thawed (SailyBio, SLB190084) and left to stand for 2 h for adhesion, and then the adherent cells were discarded. NK cells were enriched using the method provided by the NK enrichment kit (StemCell, 19055) from StemCell Technologies, and finally adjusted to a cell concentration of 1×10⁶ cells/mL with an RPMI-1640 medium (containing 5% fetal bovine serum). SK-BR3 (HER2^{high}, BCMA⁻) (i.e., a breast cancer cell strain with high HER2 expression), MDA-MB-453 (HER2^{low}) (i.e., a breast cancer cell strain with low HER2 expression), and NCI-H929 (HER2⁻, BCMA⁺) tumor cells were each digested with pre-warmed 0.25% trypsin-EDTA, and the solution obtained by the digestion was centrifuged at 400 g for 5 min. The cells were collected and counted, resuspended in an RPMI-1640 medium (containing 5% fetal bovine serum), and adjusted to a cell concentration of 2×10⁵ cells/mL. Each of the anti-CD16A/TAA bispecific antibodies was diluted with an RPMI-1640 medium (containing 5% fetal bovine serum) to make a final concentration of 100 nM, and then subjected to a 6-fold dilution to obtain 10 gradients. The cells were seeded into a plate according to the plate layout. The following components were added to each well of the experimental group: 50 µL of NK cell suspension, 50 µL of antibody sample solution, and 50 µL of tumor cell suspension. The mixture was mixed homogeneously by using a multichannel pipettor, centrifuged at 200 g for 3 min, and then mixed homogeneously. The cell culture plate was incubated in an incubator at 37 °C with 5% CO₂ for 6 h. The secretion amount of LDH (lactate dehydrogenase) in each system was determined by the assay method provided by CytoTox 96^{®} Non-Radioactive Cytotoxicity Assay (Promega, G1780).

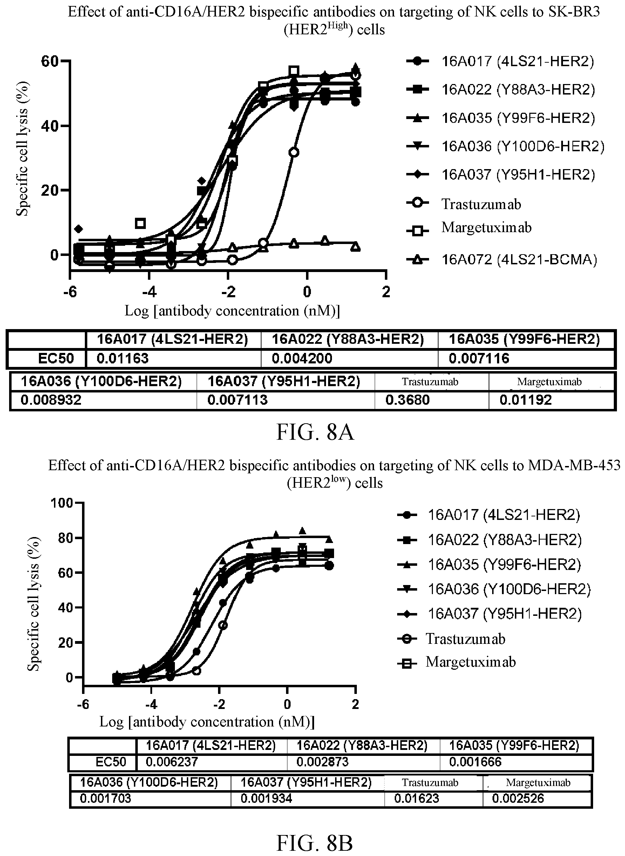

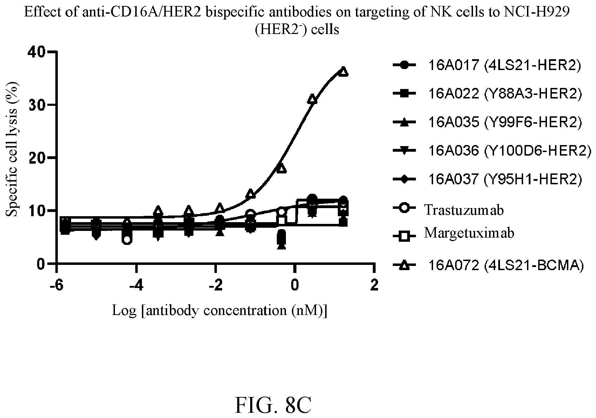

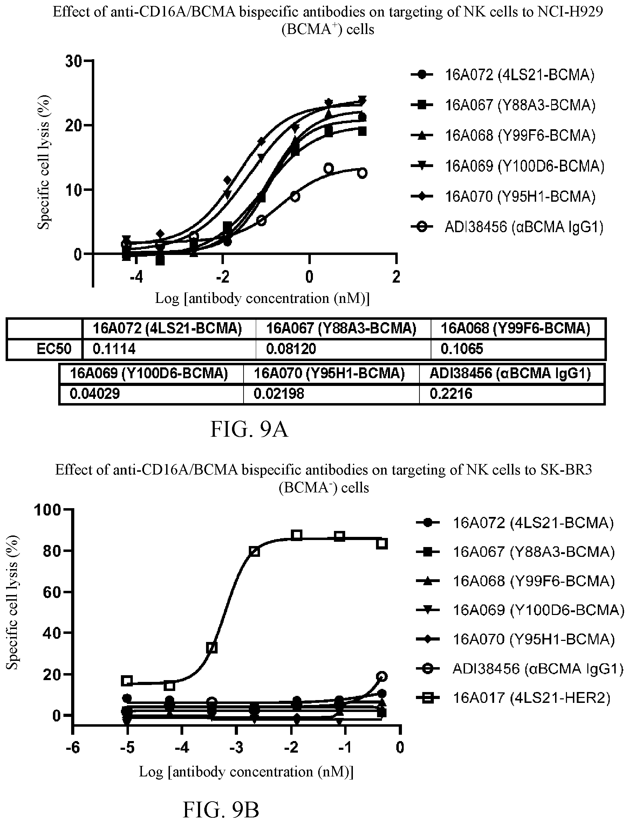

For the above experiment, as shown in FIGs. 8A-8C and FIGs. 9A-9B, the results showed that the anti-CD16A single-domain antibodies, after being assembled into bispecific antibodies with each of the two antibodies specific for tumor antigens (HER2 and BCMA), respectively, all could achieve tumor antigen-specific induction of killing of tumors by NK cells, and had a comparable or better ability to mediate NK cells to kill tumor cells as compared to the 4LS21/anti-TAA bispecific antibody and the ADCC-enhanced IgG1 antibody (margetuximab) which were used as controls.

The exemplary embodiments of the present invention have been described above. It should be understood by those skilled in the art that these contents are merely exemplary, and various other replacements, adaptations, and modifications can be made within the scope of the present invention. Therefore, the present invention is not limited to the specific embodiments listed herein.

## Claims

1. An isolated anti-CD16A single-domain antibody, comprising:
(a) 3 CDRs in an amino acid sequence of a heavy chain variable region set forth in SEQ ID NO: 1; or a CDR variant having no more than 3, no more than 2, or no more than 1 amino acid residue replacement as compared to any one of the 3 CDRs;
(b) 3 CDRs in an amino acid sequence of a heavy chain variable region set forth in SEQ ID NO: 2; or a CDR variant having no more than 3, no more than 2, or no more than 1 amino acid residue replacement as compared to any one of the 3 CDRs;
(c) 3 CDRs in an amino acid sequence of a heavy chain variable region set forth in SEQ ID NO: 3; or a CDR variant having no more than 3, no more than 2, or no more than 1 amino acid residue replacement as compared to any one of the 3 CDRs; or
(d) 3 CDRs in an amino acid sequence of a heavy chain variable region set forth in SEQ ID NO: 4; or a CDR variant having no more than 3, no more than 2, or no more than 1 amino acid residue replacement as compared to any one of the 3 CDRs.

2. The isolated anti-CD16A single-domain antibody according to claim 1, comprising:
(a) HCDR1 set forth in SEQ ID NO: 23 or a variant having no more than 3, no more than 2, or no more than 1 amino acid residue replacement of HCDR1 set forth in SEQ ID NO: 23, HCDR2 set forth in SEQ ID NO: 24 or a variant having no more than 3, no more than 2, or no more than 1 amino acid residue replacement of HCDR2 set forth in SEQ ID NO: 24, and HCDR3 set forth in SEQ ID NO: 25 or a variant having no more than 3, no more than 2, or no more than 1 amino acid residue replacement of HCDR3 set forth in SEQ ID NO: 25;
(b) HCDR1 set forth in SEQ ID NO: 26 or a variant having no more than 3, no more than 2, or no more than 1 amino acid residue replacement of HCDR1 set forth in SEQ ID NO: 26, HCDR2 set forth in SEQ ID NO: 27 or a variant having no more than 3, no more than 2, or no more than 1 amino acid residue replacement of HCDR2 set forth in SEQ ID NO: 27, and HCDR3 set forth in SEQ ID NO: 28 or a variant having no more than 3, no more than 2, or no more than 1 amino acid residue replacement of HCDR3 set forth in SEQ ID NO: 28;
(c) HCDR1 set forth in SEQ ID NO: 29 or a variant having no more than 3, no more than 2, or no more than 1 amino acid residue replacement of HCDR1 set forth in SEQ ID NO: 29, HCDR2 set forth in SEQ ID NO: 30 or a variant having no more than 3, no more than 2, or no more than 1 amino acid residue replacement of HCDR2 set forth in SEQ ID NO: 30, and HCDR3 set forth in SEQ ID NO: 31 or a variant having no more than 3, no more than 2, or no more than 1 amino acid residue replacement of HCDR3 set forth in SEQ ID NO: 31; or
(d) HCDR1 set forth in SEQ ID NO: 32 or a variant having no more than 3, no more than 2, or no more than 1 amino acid residue replacement of HCDR1 set forth in SEQ ID NO: 32, HCDR2 set forth in SEQ ID NO: 33 or a variant having no more than 3, no more than 2, or no more than 1 amino acid residue replacement of HCDR2 set forth in SEQ ID NO: 33, and HCDR3 set forth in SEQ ID NO: 34 or a variant having no more than 3, no more than 2, or no more than 1 amino acid residue replacement of HCDR3 set forth in SEQ ID NO: 34; wherein preferably, the isolated anti-CD16A single-domain antibody comprises:
(a) HCDR1 set forth in SEQ ID NO: 23, HCDR2 set forth in SEQ ID NO: 24, and HCDR3 set forth in SEQ ID NO: 25;
(b) HCDR1 set forth in SEQ ID NO: 26, HCDR2 set forth in SEQ ID NO: 27, and HCDR3 set forth in SEQ ID NO: 28;
(c) HCDR1 set forth in SEQ ID NO: 29, HCDR2 set forth in SEQ ID NO: 30, and HCDR3 set forth in SEQ ID NO: 31; or
(d) HCDR1 set forth in SEQ ID NO: 32, HCDR2 set forth in SEQ ID NO: 33, and HCDR3 set forth in SEQ ID NO: 34.

3. The isolated anti-CD16A single-domain antibody according to claim 1 or 2, comprising:
(a) a heavy chain variable region comprising an amino acid sequence of SEQ ID NO: 1 or a sequence having at least 90%, 91%, 92%, 93%, 94%, 95%, 96%, 97%, 98%, or 99% identity thereto;
(b) a heavy chain variable region comprising an amino acid sequence of SEQ ID NO: 2 or a sequence having at least 90%, 91%, 92%, 93%, 94%, 95%, 96%, 97%, 98%, or 99% identity thereto;
(c) a heavy chain variable region comprising an amino acid sequence of SEQ ID NO: 3 or a sequence having at least 90%, 91%, 92%, 93%, 94%, 95%, 96%, 97%, 98%, or 99% identity thereto; or
(d) a heavy chain variable region comprising an amino acid sequence of SEQ ID NO: 4 or a sequence having at least 90%, 91%, 92%, 93%, 94%, 95%, 96%, 97%, 98%, or 99% identity thereto.

4. A monospecific antibody molecule or an antigen-binding fragment, comprising the anti-CD16A single-domain antibody moiety according to any one of claims 1-3 and an immunoglobulin Fc region, wherein optionally, the Fc region lacks or has reduced effector functions, for example, the Fc region has a LALA mutation.

5. A multispecific antibody molecule or an antigen-binding fragment, comprising at least (i) the anti-CD16A single-domain antibody moiety according to any one of claims 1-3; (ii) a Fab fragment binding to a second antigen; and optionally (iii) an immunoglobulin Fc region located at the C-termini of the (i) and (ii), wherein optionally, the Fc region lacks or has reduced effector functions, for example, the Fc region has a LALA mutation;
wherein preferably, the multispecific antibody molecule is a bispecific antibody molecule.

6. The monospecific antibody molecule or the antigen-binding fragment according to claim 4 or the multispecific antibody molecule or the antigen-binding fragment according to claim 5, wherein the immunoglobulin is an IgG1, IgG2, or IgG4 immunoglobulin, preferably, the immunoglobulin is an IgG1 immunoglobulin, and more preferably, the immunoglobulin is a human IgG1 immunoglobulin.

7. The monospecific antibody molecule or the antigen-binding fragment or the multispecific antibody molecule or the antigen-binding fragment according to any one of claims 4-6, wherein heavy chains of the antibody molecule each comprise a protuberance and a cavity in Fc domains thereof, and the protuberance or the cavity in the Fc domain of one heavy chain can be placed at the cavity or the protuberance in the Fc domain of the other heavy chain, such that the heavy chains of the antibody molecule form a stable "knob-in-hole" association with each other.

8. The multispecific antibody molecule or the antigen-binding fragment according to any one of claims 5-7, wherein the second antigen is selected from a tumor-associated antigen, a virus-associated antigen, and a bacteria-associated antigen, wherein preferably, the tumor-associated antigen is selected from, for example, HER2, BCMA, MAGE, BAGE, MART, gp100, CD19, CD20, CD30, CD33, CD70, EpCAM, EGFR1, EGFR2, EGFR3, EGFR4, PLAP, PSMA, Claudin18.2, PD-L1, MUC1, and MUC16.

9. The multispecific antibody molecule or the antigen-binding fragment according to any one of claims 5-8, being:
i) an anti-CD16A/HER2 bispecific antibody or an antigen-binding fragment, comprising the anti-CD16A single-domain antibody moiety according to any one of claims 1-3 and an antibody fragment specifically binding to HER2,
wherein for example, the antibody fragment specifically binding to HER2 comprises 3 CDRs in an amino acid sequence of a heavy chain variable region set forth in SEQ ID NO: 15; or a CDR variant having no more than 3, no more than 2, or no more than 1 amino acid residue replacement as compared to any one of the 3 CDRs; and 3 CDRs in an amino acid sequence of a light chain variable region set forth in SEQ ID NO: 16; or a CDR variant having no more than 3, no more than 2, or no more than 1 amino acid residue replacement as compared to any one of the 3 CDRs;
for example, the antibody fragment specifically binding to HER2 comprises 3 CDRs in an amino acid sequence of a heavy chain variable region set forth in SEQ ID NO: 15 and 3 CDRs in an amino acid sequence of a light chain variable region set forth in SEQ ID NO: 16;
for example, the antibody fragment specifically binding to HER2 comprises an amino acid sequence of a heavy chain variable region set forth in SEQ ID NO: 15 or a sequence having at least 90%, 91%, 92%, 93%, 94%, 95%, 96%, 97%, 98%, or 99% identity thereto; and an amino acid sequence of a light chain variable region set forth in SEQ ID NO: 16 or a sequence having at least 90%, 91%, 92%, 93%, 94%, 95%, 96%, 97%, 98%, or 99% identity thereto;
or,
ii) an anti-CD 16A/BCMA bispecific antibody or an antigen-binding fragment,
comprising the anti-CD16A single-domain antibody moiety according to any one of claims 1-3 and an antibody fragment specifically binding to BCMA,
wherein for example, the antibody fragment specifically binding to BCMA comprises 3 CDRs in an amino acid sequence of a heavy chain variable region set forth in SEQ ID NO: 17; or a CDR variant having no more than 3, no more than 2, or no more than 1 amino acid residue replacement as compared to any one of the 3 CDRs; and 3 CDRs in an amino acid sequence of a light chain variable region set forth in SEQ ID NO: 18; or a CDR variant having no more than 3, no more than 2, or no more than 1 amino acid residue replacement as compared to any one of the 3 CDRs;
for example, the antibody fragment specifically binding to BCMA comprises 3 CDRs in an amino acid sequence of a heavy chain variable region set forth in SEQ ID NO: 17 and 3 CDRs in an amino acid sequence of a light chain variable region set forth in SEQ ID NO: 18;
for example, the antibody fragment specifically binding to BCMA comprises an amino acid sequence of a heavy chain variable region set forth in SEQ ID NO: 17 or a sequence having at least 90%, 91%, 92%, 93%, 94%, 95%, 96%, 97%, 98%, or 99% identity thereto; and an amino acid sequence of a light chain variable region set forth in SEQ ID NO: 18 or a sequence having at least 90%, 91%, 92%, 93%, 94%, 95%, 96%, 97%, 98%, or 99% identity thereto.

10. An isolated nucleic acid, encoding the anti-CD16A single-domain antibody, the monospecific antibody molecule or the antigen-binding fragment, or the multispecific antibody molecule or the antigen-binding fragment according to any one of claims 1-9.

11. A vector, comprising the nucleic acid according to claim 10, wherein preferably, the vector is an expression vector.

12. A host cell, comprising the nucleic acid according to claim 10 or the vector according to claim 11, wherein preferably, the host cell is prokaryotic or eukaryotic, more preferably, the host cell is selected from an *E. coli* cell, a yeast cell, a mammalian cell, and other cells suitable for preparing the antibody or the antigen-binding fragment, and most preferably, the host cell is a HEK293F cell.

13. A method for preparing the anti-CD16A single-domain antibody, the monospecific antibody molecule or the antigen-binding fragment, or the multispecific antibody molecule or the antigen-binding fragment according to any one of claims 1-9, comprising culturing the host cell according to claim 12 under conditions suitable for expression of the nucleic acid encoding the anti-CD16A single-domain antibody, the monospecific antibody molecule or the antigen-binding fragment, or the multispecific antibody molecule or the antigen-binding fragment according to any one of claims 1-9, and optionally recovering the anti-CD16A single-domain antibody, the monospecific antibody molecule, or the multispecific antibody molecule according to any one of claims 1-9 from the host cell or from a culture medium.

14. A pharmaceutical composition comprising the anti-CD16A single-domain antibody, the monospecific antibody molecule or the antigen-binding fragment, or the multispecific antibody molecule or the antigen-binding fragment according to any one of claims 1-9, and a pharmaceutically acceptable carrier.

15. The pharmaceutical composition according to claim 14, further comprising at least one additional active ingredient.

16. Use of the anti-CD16A single-domain antibody, the monospecific antibody molecule or the antigen-binding fragment, or the multispecific antibody molecule or the antigen-binding fragment according to any one of claims 1-9, and the pharmaceutical composition according to claims 14-15 for preparing a medicament for the treatment and/or prevention of a disease in an individual or for preparing a diagnostic tool for a disease in an individual, wherein preferably, the individual is a mammal, and more preferably, the individual is a human.

17. The use according to claim 16, for the treatment and/or prevention or diagnosis of a tumor, wherein for example, the tumor is selected from a solid tumor, a hematological cancer (e.g., leukemia, lymphoma, or myeloma, e.g., multiple myeloma), and a metastatic lesion.
